# EUROPÄISCHE PATENTSCHRIFT

(11) **EP 1 772 523 B1**
(45) Veröffentlichungstag und Bekanntmachung des Hinweises auf die Patenterteilung: **20.01.2010**
(21) Anmeldenummer: 06121808.7
(22) Anmeldetag: 05.10.2006
(51) Int. Cl.: C12Q 1/68

(54) **Polymerase-Kettenreaktionsverfahren unter Einsatz einer DNA-Polymerase mit Proofreading-Eigenschaften**
Method for polymerase chain reactions with use of a dna polymerase with proofreading properties
Méthode de réactions de polymérisation en chaîne (PCR) utilisant une ADN polymérase ayant des propriétés de correction d'erreur

(30) Priorität: 05.10.2005 DE 102005047617
(43) Veröffentlichungstag der Anmeldung: 11.04.2007
(73) Patentinhaber: Qiagen GmbH, 40724 Hilden (DE)
(72) Erfinder: Engel, Holger, 40724 Hilden (DE); Peist, Ralf, 40229 Düsseldorf (DE)

(56) Entgegenhaltungen:
- EP-A- 0 744 470
- WO-A-97/28279
- WO-A-98/28443
- SKERRA A: "PHOSPHOROTHIOATE PRIMERS IMPROVE THE AMPLICATION OF DNA SEQUENCES BY DNA POLYMERASES WITH PROOFREADING ACTIVITY" NUCLEIC ACIDS RESEARCH, OXFORD UNIVERSITY PRESS, SURREY, GB, Bd. 20, Nr. 14, 25. Juli 1992 (1992-07-25), Seiten 3551-3554, XP000363599 ISSN: 0305-1048
- YANG H-L ET AL: "High fidelity PCR with an off/on switch mediated by proofreading polymerases combining with phosphorothioate-modified primer" BIOCHEMICAL AND BIOPHYSICAL RESEARCH COMMUNICATIONS, ACADEMIC PRESS INC. ORLANDO, FL, US, Bd. 328, Nr. 1, 4. März 2005 (2005-03-04), Seiten 265-272, XP004723768 ISSN: 0006-291X
- NORONHA DE C M C ET AL: "AMPLIMERS WITH 3'-TERMINAL PHOSPHOROTHIOATE LINKAGES RESIST DEGRADATION BY VENT POLYMERASE AND REDUCE TAQ POLYMERASE MISPRIMING" PCR METHODS AND APPLICATIONS, COLD SPRING HARBOR, NY, US, Bd. 2, Nr. 2, 1. November 1992 (1992-11-01), Seiten 131-136, XP000600686 ISSN: 1054-9803

### SEQUENCE LISTING

<110> Engell, Holger Peist, Ralf
<120> METHOD FOR POLYMERASE CHAIN REACTIONS WITH USE OF A DNA POLYMERASE WITH PROOFREADING PROPERTIES
<130> PA156-EP
<150> DE102005047617.1
   <151> 2005-10-05
<160> 22
<170> FastSEQ for Windows Version 4.0
<210> 1
   <211> 22
   <212> DNA
   <213> Artificial Sequence
<220>
   <223> PCR primer sequence ERCC-1 (forward)
<400> 1
   gctgtttgat gtcctgcacg ag 22
<210> 2
   <211> 21
   <212> DNA
   <213> Artificial Sequence
<220>
   <223> PCR primer sequence ERCC-1 (reverse)
<400> 2
   gcctggcctg ggaggacgat t 21
<210> 3
   <211> 20
   <212> DNA
   <213> Artificial Sequence
<220>
   <223> Sequence of the "feed" oligonucleotide (P20-GAPDH)
<220>
   <221> modified_base
   <222> 20
   <223> 3 primer-phosphate
<400> 3
   gcgtcaaagg tggaggagtg 20
<210> 4
   <211> 30
   <212> DNA '
   <213> Artificial Sequence
<220>
   <223> Sequence of the "feed" oligonucleotide (P30-GAPDH)
<220>
   <221> modified_base
   <222> 30
   <223> 3 prime -phosphate
<400> 4
   gcgtcaaagg tggaggagtg ggtgtcgctg 30
<210> 5
   <211> 45
   <212> DNA
   <213> Artificial Sequence
<220>
   <223> Sequence of the "feed" oligonucleotide (P45-GAPDH)
<220>
   <221> modified_base
   <222> 45
   <223> 3 prime -phosphate
<400> 5
   gcgtcaaagg tggaggagtg ggtgtcgctg ttgaagtcag aggag 45
<210> 6
   <211> 22
   <212> DNA
   <213> Artificial Sequence
<220>
   <223> PCR Primer sequence (CYST 3)
<400> 6
   cccaaaccca acccatacac ac 22
<210> 7
   <211> 22
   <212> DNA
   <213> Artificial Sequence
<220>
   <223> PCR Primer sequence (CYST 5)
<400> 7
   ccttgcctta gatgtgtcgg ca 22
<210> 8
   <211> 78
   <212> DNA
   <213> Artificial Sequence
<220>
   <223> Sequences of the "feed" oligonucleotides (P78 3P)
<220>
   <221> modified_base
   <222> 78
   <223> 3 prime -phosphate
<400> 8
   gcgtcaaagg tggaggagtg ggtgtcgctg ttgaagtcat gacttcaaca gcgacaccca 60 ctcctccacc tttgacgc 78
<210> 9
   <211> 78
   <212> DNA
   <213> Artificial Sequence
<220>
   <223> Sequences of the "feed" oligonucleotides (P78 3P-thio)
<220>
   <221> modified_base
   <222> 77
   <223> phosphothioate in the backbone
<220>
   <221> modified_base
   <222> 78
   <223> 3 prime-phosphate
<400> 9
   gcgtcaaagg tggaggagtg ggtgtcgctg ttgaagtcat gacttcaaca gcgacaccca 60 ctcctccacc tttgacgc 78
<210> 10
   <211> 26
   <212> DNA
   <213> Artificial Sequence
<220>
   <223> Sequences of the "feed" oligonucleotides (N14-degAATAAA)
<220>
   <221> misc_feature
   <222> 1, 2, 3, 4, 5, 6, 7, 8, 9, 10, 11, 12, 13, 14, 17
   <223> n = A,T,C or G
<400> 10
   nnnnnnnnnn nnnnhhnddv aataaa 26
<210> 11
   <211> 27
   <212> DNA ,
   <213> Artificial Sequence
<220>
   <223> Sequences of the "feed" oligonucleotides (pA-I)
<220>
   <221> modified_base
   <222> 1, 2, 3, 4, 5, 6, 7, 8, 9, 10, 11, 12, 13, 14, 15, 16, 17, 18, 19, 20, 21
   <223> I
<400> 11
   nnnnnnnnnn nnnnnnnnnn naataaa 27
<210> 12
   <211> 35
   <212> DNA
   <213> Artificial Sequence
<220>
   <223> Sequences of the "feed" oligonucleotides (betaActp-61-3)
<220>
   <221> modified_base
   <222> 30, 31, 32, 33, 34, 35
   <223> I
<400> 12
   gtacactgac ttgagaccag ttgaataaan nnnnn 35
<210> 13
   <211> 25
   <212> DNA
   <213> Artificial Sequence
<220>
   <223> PCR Primer sequence (BACT-TM.5)
<400> 13
   tcacccacac tgtgcccatc tacga 25
<210> 14
   <211> 25
   <212> DNA
   <213> Artificial Sequence
<220>
   <223> PCR Primer sequence (BACT-TM.3)
<400> 14
   cagcggaacc gctcattgcc aatgg 25
<210> 15
   <211> 45
   <212> DNA
   <213> Artificial Sequence
<220>
   <223> Sequence of the "feed" oligonucleotide (GAP45-thio)
<220>
   <221> modified_base
   <222> 44
   <223> phosphothioate in the backbone
<220>
   <221> modified_base
   <222> 45
   <223> 3 prime -phosphate
<400> 15
   gcgtcaaagg tggaggagtg ggtgtcgctg ttgaagtcag aggag 45
<210> 16
   <211> 45
   <212> DNA
   <213> Artificial Sequence
<220>
   <223> Sequence of the "feed" oligonucleotide (G45-TP-1sp-d)
<220>
   <221> modified_base
   <222> 23
   <223> abasic spacers
<220>
   <221> modified_base
   <222> 44
   <223> phosphothioate in the backbone
<220>
   <221> modified_base
   <222> 45
   <223> 3 prime -Phosphate
<400> 16
   gcgtcaaagg tggaggagtg ggtgtcgctg ttgaagtcag aggag 45
<210> 17
   <211> 45
   <212> DNA
   <213> Artificial Sequence
<220>
   <223> Sequence of the "feed" oligonucleotide (G45-TP-2sp-d)
<220>
   <221> modified_base
   <222> 11, 23
   <223> abasic spacers
<220>
   <221> modified_base
   <222> 44
   <223> phosphothioate in the backbone
<220>
   <221> modified_base
   <222> 45
   <223> 3 prime -Phosphate
<400> 17
   gcgtcaaagg tggaggagtg ggtgtcgctg ttgaagtcag aggag 45
<210> 18
   <211> 25
   <212> DNA
   <213> Artificial Sequence
<220>
   <223> PCR primer (beta-actin forward)
<220>
   <221> modified_base
   <222> 24
   <223> phosphothioate in the backbone
<400> 18
   tcacccacac tgtgcccatc tacga 25
<210> 19
   <211> 25
   <212> DNA
   <213> Artificial Sequence
<220>
   <223> PCR primer (beta-actin reverse)
<220>
   <221> modified_base
   <222> 24
   <223> phosphothioate in the backbone
<400> 19
   cagcggaacc gctcattgcc aatgg 25
<210> 20
   <211> 23
   <212> DNA
   <213> Artificial Sequence
<220>
   <223> PCR primer (MPUC 3)
<400> 20
   gctgcttgaa gaaacgagcg gtg 23
<210> 21
   <211> 24
   <212> DNA
   <213> Artificial Sequence
<220>
   <223> PCR primer (MPUC5 +258)
<400> 21
   ctgcaccttc tggaattccg actc 24
<210> 22
   <211> 39
   <212> DNA
   <213> Artificial Sequence
<220>
   <223> Sequence of the "feed" oligonucleotides
<220>
   <221> modified_base
   <222> 38
   <223> phosphothioate in the backbone
<220>
   <221> modified_base
   <222> 39
   <223> 3 prime - phosphate
<220>
   <221> misc_feature
   <222> 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39
   <223> n = A,T,C or G
<400> 22
   nnnnnnnnnn nnnnnnnnnn nnnnnnnnnn nnnnnnnnn 39

### SEQUENCE LISTING

<110> Engell, Holger Peist, Ralf
<120> METHOD FOR POLYMERASE CHAIN REACTIONS WITH USE OF A DNA POLYMERASE WITH PROOFREADING PROPERTIES
<130> PA156-EP
<150> DE102005047617.1
   <151> 2005-10-05
<160> 22
<170> FastSEQ for Windows Version 4.0
<210> 1
   <211> 22
   <212> DNA
   <213> Artificial Sequence
<220>
   <223> PCR primer sequence ERCC-1 (forward)
<400> 1
   gctgtttgat gtcctgcacg ag 22
<210> 2
   <211> 21
   <212> DNA
   <213> Artificial Sequence
<220>
   <223> PCR primer sequence ERCC-1 (reverse)
<400> 2
   gcctggcctg ggaggacgat t 21
<210> 3
   <211> 20
   <212> DNA
   <213> Artificial Sequence
<220>
   <223> Sequence of the "feed" oligonucleotide (P20-GAPDH)
<220>
   <221> modified_base
   <222> 20
   <223> 3 prime-phosphate
<400> 3
   gcgtcaaagg tggaggagtg 20
<210> 4
   <211> 30
   <212> DNA
   <213> Artificial Sequence
<220>
   <223> Sequence of the "feed" oligonucleotide (P30-GAPDH)
<220>
   <221> modified_base
   <222> 30
   <223> 3 prime -phosphate
<400> 4
   gcgtcaaagg tggaggagtg ggtgtcgctg 30
<210> 5
   <211> 45
   <212> DNA
   <213> Artificial Sequence
<220>
   <223> Sequence of the "feed" oligonucleotide (P45-GAPDH)
<220>
   <221> modified_base
   <222> 45
   <223> 3 prime -phosphate
<400> 5
   gcgtcaaagg tggaggagtg ggtgtcgctg ttgaagtcag aggag 45
<210> 6
   <211> 22
   <212> DNA
   <213> Artificial Sequence
<220>
   <223> PCR Primer sequence (CYST 3)
<400> 6
   cccaaaccca acccatacac ac 22
<210> 7
   <211> 22
   <212> DNA
   <213> Artificial Sequence
<220>
   <223> PCR Primer sequence (CYST 5)
<400> 7
   ccttgcctta gatgtgtcgg ca 22
<210> 8
   <211> 78
   <212> DNA
   <213> Artificial Sequence
<220>
   <223> Sequences of the "feed" oligonucleotides (P78 3P)
<220>
   <221> modified_base
   <222> 78
   <223> 3 prime -phosphate
<400> 8
   gcgtcaaagg tggaggagtg ggtgtcgctg ttgaagtcat gacttcaaca gcgacaccca 60 ctcctccacc tttgacgc 78
<210> 9
   <211> 78
   <212> DNA
   <213> Artificial Sequence
<220>
   <223> Sequences of the "feed" oligonucleotides (P78 3P-thio)
<220>
   <221> modified_base
   <222> 77
   <223> phosphothioate in the backbone
<220>
   <221> modified_base
   <222> 78
   <223> 3 prime-phosphate
<400> 9
   gcgtcaaagg tggaggagtg ggtgtcgctg ttgaagtcat gacttcaaca gcgacaccca 60 ctcctccacc tttgacgc 78
<210> 10
   <211> 26
   <212> DNA
   <213> Artificial Sequence
<220>
   <223> Sequences of the "feed" oligonucleotides (N14-degAATAAA)
<220>
   <221> misc_feature
   <222> 1, 2, 3, 4, 5, 6, 7, 8, 9, 10, 11, 12, 13, 14, 17
   <223> n = A,T,C or G
<400> 10
   nnnnnnnnnn nnnnhhnddv aataaa 26
<210> 11
   <211> 27
   <212> DNA
   <213> Artificial Sequence
<220>
   <223> Sequences of the "feed" oligonucleotides (pA-I)
<220>
   <221> modified_base
   <222> 1, 2, 3, 4, 5, 6, 7, 8, 9, 10, 11, 12, 13, 14, 15, 16, 17, 18, 19, 20, 21
   <223> I
<400> 11
   nnnnnnnnnn nnnnnnnnnn naataaa 27
<210> 12
   <211> 35
   <212> DNA
   <213> Artificial Sequence
<220>
   <223> Sequences of the "feed" oligonucleotides (betaActp-6I-3)
<220>
   <221> modified_base
   <222> 30, 31, 32, 33, 34, 35
   <223> I
<400> 12
   gtacactgac ttgagaccag ttgaataaan nnnnn 35
<210> 13
   <211> 25
   <212> DNA
   <213> Artificial Sequence
<220>
   <223> PCR Primer sequence (BACT-TM.5)
<400> 13
   tcacccacac tgtgcccatc tacga 25
<210> 14
   <211> 25
   <212> DNA
   <213> Artificial Sequence
<220>
   <223> PCR Primer sequence (BACT-TM.3)
<400> 14
   cagcggaacc gctcattgcc aatgg 25
<210> 15
   <211> 45
   <212> DNA
   <213> Artificial Sequence
<220>
   <223> Sequence of the "feed" oligonucleotide (GAP45-thio)
<220>
   <221> modified_base
   <222> 44
   <223> phosphothioate in the backbone
<220>
   <221> modified_base
   <222> 45
   <223> 3 prime -phosphate
<400> 15
   gcgtcaaagg tggaggagtg ggtgtcgctg ttgaagtcag aggag 45
<210> 16
   <211> 45
   <212> DNA
   <213> Artificial Sequence
<220>
   <223> Sequence of the "feed" oligonucleotide (G45-TP-1sp-d)
<220>
   <221> modified_base
   <222> 23
   <223> abasic spacers
<220>
   <221> modified_base
   <222> 44
   <223> phosphothioate in the backbone
<220>
   <221> modified_base
   <222> 45
   <223> 3 prime -Phosphate
<400> 16
   gcgtcaaagg tggaggagtg ggtgtcgctg ttgaagtcag aggag 45
<210> 17
   <211> 45
   <212> DNA
   <213> Artificial Sequence
<220>
   <223> Sequence of the "feed" oligonucleotide (G45-TP-2sp-d)
<220>
   <221> modified_base
   <222> 11, 23
   <223> abasic spacers
<220>
   <221> modified_base
   <222> 44
   <223> phosphothioate in the backbone
<220>
   <221> modified_base
   <222> 45
   <223> 3 prime -Phosphate
<400> 17
   gcgtcaaagg tggaggagtg ggtgtcgctg ttgaagtcag aggag 45
<210> 18
   <211> 25
   <212> DNA
   <213> Artificial Sequence
<220>
   <223> PCR primer (beta-actin forward)
<220>
   <221> modified_base
   <222> 24
   <223> phosphothioate in the backbone
<400> 18
   tcacccacac tgtgcccatc tacga 25
<210> 19
   <211> 25
   <212> DNA
   <213> Artificial Sequence
<220>
   <223> PCR primer (beta-actin reverse)
<220>
   <221> modified_base
   <222> 24
   <223> phosphothioate in the backbone
<400> 19
   cagcggaacc gctcattgcc aatgg 25
<210> 20
   <211> 23
   <212> DNA
   <213> Artificial Sequence
<220>
   <223> PCR primer (MPUC 3)
<400> 20
   gctgcttgaa gaaacgagcg gtg 23
<210> 21
   <211> 24
   <212> DNA
   <213> Artificial Sequence
<220>
   <223> PCR primer (MPUC5 +258)
<400> 21
   ctgcaccttc tggaattccg actc 24
<210> 22
   <211> 39
   <212> DNA
   <213> Artificial Sequence
<220>
   <223> Sequence of the "feed" oligonucleotides
<220>
   <221> modified_base
   <222> 38
   <223> phosphothioate in the backbone
<220>
   <221> modified_base
   <222> 39
   <223> 3 prime - phosphate
<220>
   <221> misc_feature
   <222> 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39
   <223> n = A,T,C or G
<400> 22
   nnnnnnnnnn nnnnnnnnnn nnnnnnnnnn nnnnnnnnn 39

## Beschreibung

Die vorliegende Erfindung betrifft ein Polymerase-Kettenreaktions-Verfahren, bei welchem eine DNA-Polymerase eingesetzt wird, die eine Proofreading-Eigenschaft aufweist.

Das Polymerase-Kettenreaktions-Verfahren ("Polymerase Chain Reaction" oder "PCR") ist ein gentechnologisch angewendetes Verfahren, bei dem es gelingt, einige wenige Moleküle einer beliebigen DNA-Sequenz in kurzer Zeit *in vitro* um Faktoren von 10⁶ bis 10⁸ zu vermehren. Üblicherweise benötigt man dazu zwei synthetisch hergestellte Oligodesoxynukleotid-Primer mit ca. 15 bis 30 Nukleotiden Länge, deren Sequenzen zu den Anfangs- und Endsequenzen der Schwesterstränge der zu vermehrenden ("amplifizierenden") DNA komplementär sind, ein Gemisch aus 4'-Desoxynukleotid-5'-triphosphaten, sowie eine thermostabile DNA-Polymerase, die zumindest eine kurzzeitige Erwärmung auf 95°C ohne Funktionseinbuße verträgt.

Häufig werden bei der PCR auch thermostabile DNA-Polymerasen eingesetzt, die eine "Proofreading"-Eigenschaft aufweisen (sog. "High-Fidelity DNA-Polymerasen"). Diese High-Fidelity DNA-Polymerasen zeichnen sich durch eine geringe Fehlerrate bzw. hohe Genauigkeit bei der DNA Synthese aus. Bei der Verwendung solcher High Fidelity DNA-Polymerasen treten jedoch oft Probleme dahingehend auf, daß nur eine sehr geringe Ausbeute an PCR-Produkt erreicht wird, bis hin zum Totalausfall der PCR (d.h. es entsteht überhaupt kein PCR-Produkt). Dieses Problem tritt insbesondere dann auf, wenn nur geringe Mengen an Template-Nukleinsäure oder geringe Primer-Konzentrationen zur Verfügung stehen. Der Grund dafür ist sehr wahrscheinlich der Abbau der Template-Nukleinsäure und der Primer durch die Proofreading-Aktivität der High Fidelity DNA Polymerase. Dieser Verlust an Template-Nukleinsäure und Primern führt dann zu einer dramatischen Reduktion der Amplifikationsrate, was wiederum eine verringerte Ausbeute an PCR-Produkt oder sogar den Totalausfall der PCR zur Folge hat.

Es wurde gezeigt, daß PCR-Primer, die eine Phosporo-Phosphothioat-Bindung an ihrem 3'-Ende im DNA-Rückgrat besitzen, gegen Abbau durch die 3'-5'-Exonuklease-Aktivität der eingesetzten High-Fidelity DNA-Polymerase geschützt sind. Bei Verwendung solcher Primer kann die Ausbeute und Zuverlässigkeit der High Fidelity PCR verbessert werden, siehe. z.B. de Noronha, C. M. und Mullins J. I., Amplimers with 3'-terminal phosphoroPhosphothioat linkages resist degradation by vent polymerase and reduce Taq polymerase mispriming, PCR Methods Appl, 1992. 2(2), 131-136; und Skerra, A., PhosphoroPhosphothioat primers improve the amplification of DNA sequences by DNA polymerases with proofreading activity, Nucleic Acids Res, 1992, 20(14), 3551-3554. Der Effekt solcher Primer auf die Sensitivität und Ausbeute der High Fidelity PCR wurde auch im Rahmen der Experimente für diese Erfindung getestet. Es wurde deutlich, daß die Verwendung solcher Primer einen weitaus geringeren Effekt auf Sensitivität und Ausbeute der High Fidelity PCR besitzt als das der vorliegenden Erfindung zugrundeliegende Prinzip.

Es gibt nun die oben beschriebene Beobachtung, daß High Fidelity DNA-Polymerasen Schwierigkeiten bei der Reproduzierbarkeit sowie der Sensitivität der PCR-Reaktionen bereiten, speziell wenn geringe Template-Mengen verwendet werden. Vielfach kann bei suboptimalen Bedingungen die PCR nicht erfolgreich durchgeführt werden. Eine High Fidelity PCR erfordert dadurch viel Optimierung, da es in viel stärkerem Ausmaß als z.B. bei PCR mit Taq-Polymerase auf die eingesetzte Start-Template-Menge ankommt.

Im Hinblick auf die oben beschriebene Probleme des Standes der Technik war es daher die Aufgabe der vorliegenden Erfindung, ein Polymerase-Kettenreaktions-Verfahren zur Verfügung zu stellen, bei dem eine High Fidelity DNA-Polymerase bei geringem Template-Nukleinsäure-Ausgangsmaterial eingesetzt werden kann, und dennoch eine zufriedenstellende Ausbeute an PCR-Produkt erzielt wird.

Diese Aufgabe löst die Erfindung durch das im unabhängigen Anspruch 1 angegebene Verfahren sowie durch den in Anspruch 67 angegebenen Kit. Weitere vorteilhafte Aspekte, Ausgestaltungen und Details der Erfindung ergeben sich aus den abhängigen Ansprüchen, der Beschreibung, den Beispielen und der Zeichnung.

Die vorliegende Erfindung gibt somit ein Polymerase-Kettenreaktions-Verfahren an, bei dem eine Template-Nukleinsäure, mindestens ein Primer, Deoxyribonukleosidtriphosphate sowie eine Polymerase mit Proofreading-Aktivität eingesetzt werden, wobei das Verfahren dadurch gekennzeichnet ist, daß bei der Polymerase-Kettenreaktion mindestens ein Zielsubstrat zugesetzt wird. Als Zielsubstrat ist jedes Molekül geeignet, das die 3'-5'-Exonukleaseaktivität der verwendeten DNA-Polymerase reduziert oder im Optimalfall blockiert. Man könnte auch sagen, daß die 3'-5'-Exonukleaseaktivität der verwendeten DNA-Polymerase auf das Zielsubstrat ab- oder umgelenkt wird.

Die PCR-Produktausbeute sowie die Sensitivität und der Verläßlichkeit der PCR unter Einsatz einer High Fidelity DNA Polymerase wird somit erfindungsgemäß durch eine gerichtete Ablenkung überzähliger (unbeschäftigter) High Fidelity DNA-Polymerase Moleküle auf ein der Reaktion zugesetztes Zielsubstrat (auch als "Futter" bezeichnet) erreicht. Die Begriffe "Zielsubstrat" und "Futter" werden im Rahmen der vorliegenden Erfindung synonym verwendet. Durch dieses "Ablenken" auf ein zugesetztes Substrat wird der Abbau der Primer und/oder der Template-DNA durch die Proofreading-Aktivität der High Fidelity DNA-Polymerase derart reduziert, daß eine verläßliche Durchführung der PCR-Reaktion unter Bedingungen möglich ist, die ohne Zusatz des "Futters" nur eine geringe PCR-Produktausbeute oder sogar den Totalausfall der PCR liefern würden. Besonders vorteilhaft ist dabei, daß eine erfolgreiche PCR bei Verwendung des "Futters" mit deutlich variableren Mengen an Start-Template-Nukleinsäure möglich ist. Hierdurch werden aufwendige Optimierungen der experimentellen Bedingungen deutlich reduziert, was auch den Aufwand an Zeit und Material erheblich verringert.

Das zugesetzte Zielsubstrat zeichnet sich dadurch aus, daß es in der Lage ist, unter PCR-Bedingungen doppelsträngige Strukturen auszubilden, die als Bindungsstelle für die High Fidelity DNA-Polymerase dienen können. Das zugesetzte Zielsubstrat sollte die PCR-Reaktion nicht stören und idealerweise zusätzlich so formuliert sein, daß es überhaupt nicht an der PCR-Reaktion teilnimmt (d.h. nicht amplifiziert wird). Eine Störung der Amplifikation ist beispielsweise dann nicht gegeben, wenn keine allgemein auftretenden Nebenprodukte in störender Menge entstehen oder die Futter-DNA per se detektierbar ist. Eine störende Menge ist im allgemeinen dann erreicht, wenn die generierten Nebenprodukte bei der verwendeten Analysemethode der PCR-Produkte als Hintergrund nachweisbar werden oder einen störenden Einfluss bei nachgeschalteten Anwendungen, wie z.B. Klonierungen, *in vitro* Transkription/Translation oder Mutagenese, zeigen.

Technische Lösungen für das zugesetzte Substrat (Zielsubstrat) sind insbesondere einzelsträngige, lineare Oligonukleotide, Haarnadel-Oligonukleotide, sowie RNA- und DNA-Moleküle. Beispiele für mögliche Formulierungen werden nachfolgend näher beschrieben.

Bei Verwendung von ProofStart DNA-Polymerase (QIAGEN GmbH, Hilden, Deutschland) zeigte sich, daß die Verwendung einer Konzentration von 1 µM für jeden der beiden benötigten Primer die High Fidelity PCR mit ProofStart DNA-Polymerase (QIAGEN GmbH, Hilden, Deutschland) stabiler macht. Dies ist eine höhere Konzentration, als für eine Standard-PCR mit Taq-Polymerase empfohlen wird (typischerweise 0.2-0.4 µM). Ohne auf diese Theorie festgelegt werden zu wollen gehen die Erfinder der vorliegenden Anmeldung davon aus, daß die höhere Robustheit mit einer Konzentration von 1 µM Primer darauf beruhen könnte, daß ein Teil der Primer durch die ProofStart DNA-Polymerase abgebaut wird.

Das erfindungsgemäße Verfahren ist mit allen marktüblichen High Fidelity DNA-Polymerasen durchführbar. Diese Enzyme können optional eine "Hot-Start" Funktion besitzen oder auch ohne "Hot-Start" eingesetzt werden. Der Hot-Start kann dabei mittels aller marktüblichen Verfahren erfolgen, einschließlich der Blockierung der Enzymaktivität durch Antikörper oder durch 'chemische Modifikationen (vergl. z.B. US-A-5,677,152, EP-A-0 771 870 und EP-A-0 962 526).

Erfindungsgemäß wird auch ein Kit zur Verfügung gestellt. Ein solcher Kit enthält mindestens ein Gefäß mit einem Zielsubstrat bzw. "Futter" im Sinne dieser Erfindung. In einer bevorzugten Form enthält dieser Kit zusätzlich eine High Fidelity DNA-Polymerase (DNA-Polymerase mit Proofreading-Aktivität), einen oder mehrere PCR-Puffer, dNTPs, eine Lösung mit Mg²⁺-Ionen und/oder ein oder mehrere geeignete PCR-Additiv(e), wie Betain, Polyethylenglykol (PEG), Dextran, Glyzerin, Dimethylsulfoxid (DMSO), Bovine Serum Albumin (BSA), einzelsträngiges, DNA-bindendes Protein (single-strand DNA-binding-protein), (nicht-ionische) Detergenzien oder andere geeignete Substanzen. Jede Komponente kann entweder einzeln oder als Mischung von zwei oder mehreren Komponenten formuliert sein. Vorzugsweise ist der erfindungsgemäße Kit dafür vorgesehen, das erfindungsgemäße Verfahren durchzuführen. Daher gilt alles, was in Bezug auf das Verfahren .gesagt wird, ebenso für den Kit.

Für die Ablenkung der High Fidelity DNA-Polymerase sind verschiedene technische Lösungen denkbar, die im Detail nachfolgend beschrieben werden und deren Funktion in den Beispielen mit Daten belegt wird. Die einzelnen Aspekt der verschiedenen Ausführungsformen sind beliebig austauschbar, soweit dies technisch sinnvoll machbar ist.

Eine erste bevorzugte Ausführungsform der Erfindung betrifft den Zusatz von einzelsträngigen Oligonukleotiden als Zielsubstrat bzw. "Futter" zu einer PCR.

Die erfindungsgemäß einsetzbaren einzelsträngigen Oligonukleotide zeichnen sich dadurch aus, daß sie in der Lage sind, unter PCR-Bedingungen Doppelstränge auszubilden, die als Bindungsstelle für die High Fidelity DNA-Polymerase dienen können. Das zugesetzte "Futter"-Oligonukleotid sollte im bevorzugten Fall zusätzlich so formuliert sein, daß es nicht in unerwünschter Weise an der PCR-Reaktion teilnimmt. Denkbare technische Lösungen sowie grundsätzliche Eigenschaften des zugesetzten "Futter"-Oligonukleotids werden im Folgenden näher beschrieben.

Erwünscht sein kann z.B. die Verhinderung der 3'-Verlängerung des Zielsubstrats bzw. des "Futter"-Oligonukleotids durch die verwendete DNA-Polymerase. Das "Futter"-Oligonukleotid sollte in der bevorzugten Ausführungsform nicht durch die DNA-Polymerase verlängerbar sein, d.h. nicht selbst als Primer dienen. Dies kann durch Modifikation des 3'-Endes des Oligonukleotids erreicht werden, so daß dieses nicht mehr durch die High Fidelity DNA-Polymerase verlängert werden kann. Hierfür sind verschiedene Lösungen denkbar, die bereits während der Synthese des Zielsubstrats eingebaut werden können. Zu diesen Lösungen gehört der Einbau von Didesoxy-Nukleotiden, inversen Basen, RNA, abasischen Sites, Spacern, Farbstoffen, Quencher-Resten wie z.B. Black Hole Quencher, Dabcyl, Minor-Groove-Binder, modifizierten Basen wie z.B. Super-Basen oder halogenierte Basen, oder Basen-Analoga, sowie alle anderen denkbaren Modifikationen am Zucker-Rückgrat, den Basen sowie zusätzliche Seitengruppen, welche die katalytische Fähigkeit der High Fidelity DNA-Polymerase zur DNA-Synthese inhibieren. Die bevorzugte Lösung für den Schutz vor Verlängerung der Primer ist der Einbau einer 3' Phosphat-Gruppe anstatt der benötigten 3'-OH-Gruppe in ein DNA-Oligonukleotid.

Eine weitere Möglichkeit betrifft die Verhinderung der 3'-Verkürzung des Zielsubstrats durch DNA-Rückgrat Modifikationen. Bedingt durch ihre 3'-5'-Exonuklease-Aktivität ist die High Fidelity DNA-Polymerase in der Lage, einen Primer am 3'-Ende zu verkürzen, wodurch eine Modifikation zur Verhinderung der 3'-Verlängerung unerwünschterweise entfernt werden könnte. Diese 3'-5'-exonukleolytische Verkürzung kann durch eine Veränderung oder mehrere Veränderungen im Rückgrat der DNA verhindert werden. Bevorzugte Lösung dafür ist der Einbau mindestens eines Phosphothioats anstelle eines Phosphats im Zucker-Phosphat-Rückgrat des DNA-Oligonukleotids. Um eine 3'-5'-exonukleolytische Verkürzung komplett zu unterbinden reicht es aus, das Phosphat im Rückgrat zwischen der letzten und der vorletzten 3'-Base des ,DNA-Oligonukleotids durch ein Phosphothioat zu ersetzen. Ein entsprechender Ersatz ist im folgenden Formelschema gezeigt (Struktur einer DNA mit Phosphothioat-Modifikationen im Rückgrat).

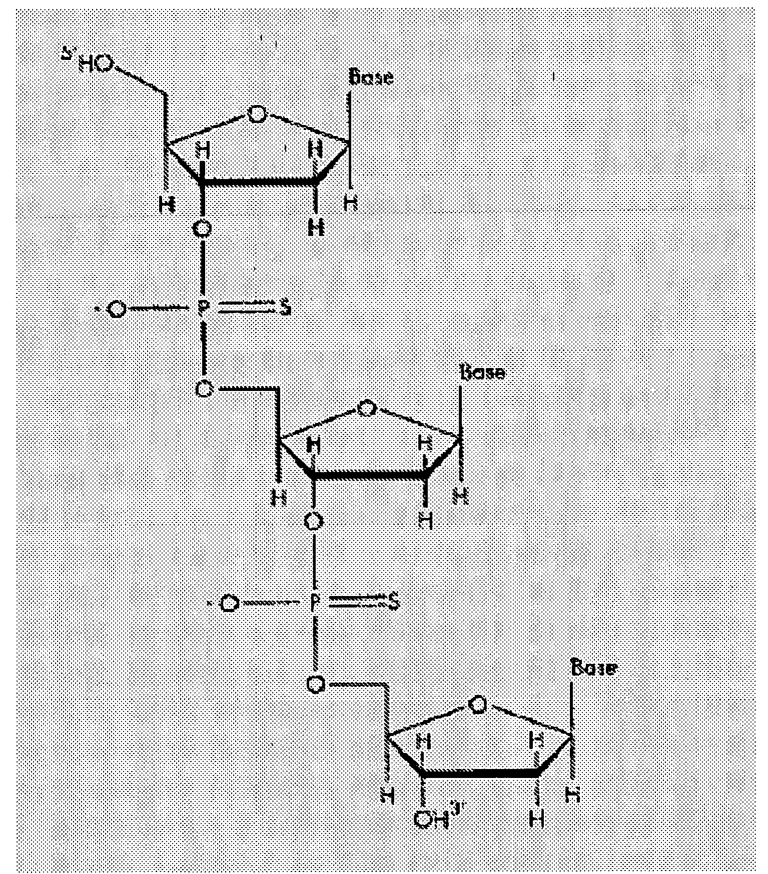

Eine weitere Möglichkeit zur Verhinderung der exonukleolytischen Verkürzung kann durch die Verwendung von Peptide Nucleic Acids (PNA) erreicht werden.

Ein wesentlicher Bestandteil dieser Erfindung ist, daß die als "Futter" eingesetzte Nukleinsäure während der PCR-Reaktion in der Lage ist, doppelsträngige Strukturen mit sich selbst oder anderen vorhandenen Nukleinsäuremolekülen auszubilden.

Ein weiterer Aspekt der vorliegenden Erfindung betrifft die Basensequenz des Zielsubstrats. Für die Basensequenz des als "Futter" verwendeten Oligonukleotids sind verschiedene Lösungen möglich. Erfolgreich getestet wurden sowohl Oligonukleotide, die zu einer definierten Zielsequenz in der verwendeten Template-Nukleinsäure komplementär sind, als auch solche mit mehr oder weniger zufälliger Sequenz (sog. "Random-Oligonukleotide") enthaltend die Basen Adenin (A), Cytosin (C), Guanin (G) und Thymin (T). Auch können Oligonukleotide eingesetzt werden, die einzeln oder in Kombination mit A, C, G, T eine oder mehrere universelle Basen (wie z.B. Inosin, 3-Nitropyrrol oder 5-Nitro-indol), ein oder mehrere Uracil, Methyl-Cytosin, Basen-Analoga oder modifizierte Basen enthalten, sowie Homo-Oligomere bestehend aus einer Folge der gleichen Base (wie z.B. Oligo-dT), einer universellen Base, einer modifizierten Base oder einem Basen-Analogon.

Ein wesentlicher Bestandteil dieser Erfindung ist, daß komplementäre Bindungsstellen für die als "Futter" eingesetzten Oligonukleotide in der PCR-Reaktion vorliegen. Diese Bindungsstellen können sowohl auf der als Template eingesetzten Nukleinsäure als auch auf dem als "Futter" eingesetzten Oligonukleotid selbst vorliegen. Diese Eigenschaft wird z.B. von Random-Oligonukleotiden erfüllt, da diese auf jeder denkbaren Template-Nukleinsäure Bindungstellen finden und zusätzlich auch mit anderen Random-Oligonukleotiden Doppelstränge ausbilden können.

Die Bindungsstellen, an denen ein DNA-Doppelstrang ausgebildet wird, müssen nicht perfekt komplementär sein, sondern unter typischen PCR Bedingungen, speziell im Annealing-Schritt, dürfen auch Bindungen mit einer Basen-Fehlpaarung oder mehreren Basen-Fehlpaarungen auftreten. Gerade bei Oligonukleotiden mit einer Länge von mehr als 20 Basen ist das Auftreten von Basen-Fehlpaarungen sehr wahrscheinlich.

Das "Futter"-Oligonukleotid sollte vorzugsweise eine Länge aufweisen, bei der unter PCR-Bedingungen die Bindung an einen geeigneten DNA-Gegenstrang möglich ist. Das Oligonukleotid muss demnach mindestens eine Länge aufweisen, die eine effiziente Bindung in einem PCR Puffer bei ca. 40°C-90°C erlaubt. Typischerweise sollte eine Bindung im Annealing-Schritt der PCR erfolgen, welcher meist in einem Temperaturbereich von ca. 45°C-70°C durchgeführt wird. Daraus ergibt sich eine ,bevorzugte Länge des Oligonukleotids von ca. 10 bis ca. 100 Basen, mehr bevorzugt ca. 12 bis ca. 80 Basen, insbesondere ca. 20 bis 45 Basen. Die zu wählende bevorzugte Länge hängt z.B. von der Sequenz, dem GC-Gehalt und eventuellen sonstigen Modifikationen des Oligonukleotids ab.

Die bevorzugte effektive Konzentration des "Futter"-Oligonukleotids liegt in einem Bereich von ca. 0,1 bis ca. 20 µM, mehr bevorzugt ca. 0,2 bis ca. 2,5 µM, insbesondere ca. 0,5 bis ca. 1,5 µM. Die optimale Konzentration wird durch die sonstigen Eigenschaften des Oligonukleotids, wie z.B. Länge und Basensequenz, mitbestimmt, da durch die Konzentration ebenfalls die Effizient der Bindung an den DNA-Gegenstrang beeinflußt wird. Die bevorzugte Konzentration liegt beispielsweise für 20 bis 45 Basen enthaltende Oligonukleotide mit definierter Sequenz oder zufälliger Sequenz (Random-Oligonukleotide) zwischen 0,2 und 2,5 µM.

Außerdem hängt die bevorzugte effektive Konzentration des Zielsubstrats auch von der Konzentration der verwendeten High Fidelity DNA-Polymerase ab. Geht man dabei davon aus, dass 1 U High Fidelity DNA-Polymerase ungefähr 1 pmol Polymerase-Molekülen entsprechen, dann ergibt sich ein bevorzugtes Verhältnis High Fidelity DNA-Polymerase Moleküle zu "Futter"-Oligonukleotid zwischen 1:1 und 1:1000, mehr bevorzugt zwischen 1:1 und 1:500 und insbesondere zwischen 1:1 und 1:200. Generell läßt sich sagen, daß bei einem kurzen Futter-Oligonukleotid das Verhältnis eher größer und bei einem längeren Futter-Oligonukleotid das Verhältnis eher kleiner sein sollte. In jedem Fall ist darauf zu achten, daß die Anzahl der Futter-Oligonukleotidmoleküle bzw. das Verhältnis zwischen High Fidelity DNA-Polymerase Molekülen zu "Futter"-Oligonukleotidmolekülen so gewählt wird, daß die Amplifikation nicht inhibiert wird.

Das 5'-Ende des "Futter"-Oligonukleotids kann unmodifiziert sein oder zusätzlich eine Modifikation tragen. Eine 5'-Modifikation ist im wesentlichen unkritisch im Sinne dieser Erfindung, solange dadurch die Bindungseigenschaften des Oligonukleotids zur Template-DNA und die Bindung der High Fidelity DNA-Polymerase nicht behindert werden. Mögliche Modifikationen umfassen C-Linker (e.g. C3-, C6-, C9-, C12-, C18-Linker), 5'-Phosphat-Modifikationen, Amino-Modifikationen, Haptene incl. DIG, Fluorescein, Biotin, Fluoreszenz-Farbstoffe, Quencher-Reste, Thiol-Labels und sonstige bekannte 5'- Modifikationen, welche die Bindung des Oligonukleotids und der High Fidelity DNA Polymerase an der Template-Nukleinsäure nicht be- oder verhindern.

Interne Modifikation des "Futter"-Oligonukleotids sind ebenfalls möglich und können vorteilhaft im Sinne dieser Erfindung sein. Interne Modifikationen können die Basen oder das Zucker-Phosphat-Rückgrat betreffen. Es wurde bereits oben im Abschnitt unter dem Stichwort Verhinderung der 3'-Verkürzung durch DNA-Rückgrat Modifikationen auf Modifikationen des DNA Rückgrats eingegangen. Neben dem dort erwähnten Austausch von Phosphat gegen Phosphothioat an einer, mehreren oder allen Positionen des DNA-Rückgrats sind alle bekannten Veränderungen des Rückgrats denkbar, solange dadurch die Bindung der High Fidelity DNA-Polymerase an der Template-Nukleinsäure nicht komplett verhindert wird. Weiterhin ist der Einbau von abasischen Sites durch Abstandshalter ("Spacer") möglich, oder es können andere Modifikationen an Basen, Rückgrat oder Seitenketten eingeführt werden. Besonders erwähnt seien hier die Locked Nucleic Acids, die sich durch einen modifizierten Zucker auszeichnen.

Eine zweite grundlegende Ausführungsform der vorliegenden Erfindung betrifft den Einsatz von doppelsträngigen Oligonukleotiden (auch "Haarnadel-Oligonukleotide" genannt) als Zielsubstrat.

Diese doppelsträngigen Oligonukleotide zeichnen sich dadurch aus, daß sie durch eine mit sich selbst komplementäre Haarnadelstruktur einen DNA-Doppelstrang ausbilden können. Die Länge und Basensequenz der Haarnadel-Oligonukleotide sind dabei so gewählt, daß diese mindestens während des Annealing Schritts der PCR als DNA-Doppelstrang vorliegen. Das zugesetzte "Futter"-Oligonukleotid sollte im bevorzugten Fall zusätzlich so formuliert sein, daß es in keiner unerwünschten Weise an der PCR-Reaktion teilnimmt. Mögliche Lösungen sowie bevorzugte grundsätzliche Eigenschaften des zugesetzten "Futter"-Oligonukleotids werden nachfolgend beschrieben.

Das "Futter"-Oligonukleotid sollte in seiner bevorzugten Ausführungsform nicht durch die DNA-Polymerase verlängerbar sein, d.h. nicht selbst als Primer dienen können. Dies kann durch eine Modifikation des 3'-Endes des Oligonukleotids erreicht werden, so daß dieses nicht mehr durch die High Fidelity DNA-Polymerase verlängert werden kann. Dies kann auf verschiedenen Wegen erreicht werden. Die Modifikationen werden vorzugsweise bereits während der DNA-Synthese eingebaut. Zu diesen möglichen, geeigneten Modifikationen gehört der Einbau von Didesoxy-Nukleotiden, inversen Basen, RNA, abasischen Sites, Spacern, Farbstoffen, Quencher-Resten wie z.B. Black Hole Quencher, Dabcyl, Minor-Groove-Binder, modifizierten Basen wie z.B. Super-Basen oder halogenierte Basen, oder Basen-Analoga, und alle anderen denkbaren Modifikationen am Zucker-Rückgrat oder den Basen des "Futter"-Oligonukleotids sowie zusätzliche Seitengruppen, welche die katalytische Fähigkeit der High Fidelity DNA-Polymerase zur DNA-Synthese inhibieren. Die bevorzugte Lösung für den Schutz vor Verlängerung des Oligonukleotids ist der Einbau einer 3'-Phosphat-Gruppe anstatt der von der Polymerase benötigten 3'-OH-Gruppe in das DNA-Oligonukleotid (siehe oben).

Auch ist eine Verhinderung der 3'-Verkürzung des Oligonukleotids durch DNA-Rückgrat-Modifikation(en) möglich. Bedingt durch ihre 3'-5'-Exonuklease Aktivität ist die High Fidelity DNA-Polymerase in der Lage, einen solchen Primer am 3'-Ende zu verkürzen, wodurch die Modifikation zur Verhinderung der 3'-Verlängerung unerwünschterweise entfernt werden könnte. Diese 3'-5'-exonukleolytische Verkürzung kann durch eine Veränderung oder mehrere Veränderungen im Rückgrat der DNA verhindert werden. Bevorzugte Lösung dafür ist der Einbau mindestens eines Phosphothioats anstelle eines Phosphats im Zucker-Phosphat-Rückgrat des DNA-Oligonukleotids. Um eine 3'-5'-exonukleolytische Verkürzung komplett zu unterbinden reicht es aus, das Phosphat im Rückgrat zwischen der letzten und vorletzten 3'-Base des DNA-Oligonukleotids durch ein Phosphothioat zu ersetzen. Eine weitere Möglichkeit zur Verhinderung der exonukleolytischen Verkürzung kann durch die Verwendung von Peptide Nucleic Acids (PNA) erreicht werden.

Für die Basensequenz des als "Futter" verwendeten DNA-Oligonukleotids sind ebenfalls verschiedene Lösungen möglich. Für Haarnadel-Oligonukleotide ist die bevorzugte Lösung die Verwendung von definierten Zielsequenzen, die eine mit sich selbst komplementäre Haarnadel-Struktur ausbilden können. Diese Oligonukleotide können verschiedene Modifikationen sowohl in der Basensequenz als auch im Rückgrat der DNA tragen. So kann die Haarnadel Struktur eine Basen-Fehlpaarung oder mehrere Basen-Fehlpaarungen aufweisen oder abasische Sites enthalten. Es können auch universelle oder modifizierte Basen in der DNA vorhanden sein.

Ein wesentlicher Aspekt der vorliegenden Erfindung ist, daß die als "Futter"-Oligonukleotid eingesetzten Oligonukleotide während der PCR-Reaktion in der Lage sind, doppelsträngige Strukturen auszubilden, die als Bindungsstelle für die High Fidelity DNA-Polymerase dienen 0 können. Diese Bindungsstellen können sowohl durch Bindung des "Futter"-Oligonukleotids auf der als Template eingesetzten Nukleinsäure entstehen als auch auf dem als "Futter" eingesetzten Haarnadel-Oligonukleotid selbst vorliegen.

Die Bindungsstellen, an denen ein DNA-Doppelstrang ausgebildet wird, müssen nicht perfekt komplementär sein, sondern es können auch Bindungen mit einer Basen-Fehlpaarung oder mehreren Basen-Fehlpaarungen auftreten. Ein "Futter"-Oligonukleotid im Sinne der Erfindung sollte zumindest im Annealing-Schritt der PCR einen DNA-Doppelstrang ausbilden können. Das Annealing wird meist in einem Temperaturbereich von ca. 45°C-70°C durchgeführt. Daraus ergibt sich eine bevorzugte Länge des selbstkomplementären Bereichs der Haarnadelstruktur des Oligonukleotids von ca. 10 bis ca. 100 Basen, mehr bevorzugt von ca. 12 bis ca. 80 Basen, insbesondere von ca. 20 bis ca. 45 Basen. Der selbstkomplementäre Bereich ist dabei ein einzelsträngiger Abschnitt des betreffenden Oligonukleotids, der mit einer mehr oder weniger komplementären Struktur des selben Oligonukleotids zu einem Doppelstrang hybridisieren kann. Die zu wählende bevorzugte Länge hängt u.a. von der Sequenz, dem GC-Gehalt und eventuellen sonstigen Modifikationen des Oligonukleotids ab.

Das "Futter"-Oligonukleotid weist vorzugsweise eine Länge auf, bei der unter PCR-Bedingungen die Bildung eines-DNA-Doppelstrangs oder die Bindung an einen geeigneten DNA-Gegenstrang möglich ist. Das Oligonukleotid sollte demnach mindestens eine Länge aufweisen, die eine effiziente Bindung in einem PCR-Puffer bei ca. 40°C-90°C erlaubt. Typischerweise sollte eine Bindung im Annealing-Schritt der PCR erfolgen, welcher meist in einem Temperaturbereich von ca. 45°C-70°C durchgeführt wird. Daraus ergibt sich eine bevorzugte Länge des selbstkomplementären Bereichs der Haarnadelstruktur des Oligonukleotids von ca. 10 bis ca. 100 Basen, mehr bevorzugt von ca. 12 bis ca. 80 Basen, insbesondere von ca. 20 bis ca. 45 Basen. Die zu wählende bevorzugte Länge hängt u.a. von der Sequenz, dem GC-Gehalt und eventuellen sonstigen Modifikationen des Oligonukleotids ab.

Die effektive Konzentration des "Futter"-Oligonukleotids liegt bevorzugt in einem Bereich von ca. 0,05 bis 20 µM, beispielsweise 0,2 bis 10 µM, mehr bevorzugt ca. 0,2 bis 2,5 µM Die optimale Konzentration wird durch die sonstigen Eigenschaften des Oligonukleotids, wie z.B. Länge und Basensequenz, mitbestimmt. Die bevorzugte Konzentration liegt für 20 bis 45 Basen umfassende Oligonukleotide mit definierter Sequenz oder zufälliger Sequenz (Random-Oligonukleotide), zwischen 0,2 und 2,5 µM. Zusätzlich hängt die effektive Konzentration auch von der Konzentration der High Fidelity DNA-Polymerase ab. Geht man dabei davon aus, dass 1, U High Fidelity DNA-Polymerase ungefähr 1 pmol Polymerase-Moleküle entsprechen, dann ergibt sich ein bevorzugtes Verhältnis High Fidelity DNA Polymerase Molekülen zu "Futter"-Oligonukleotid zwischen 1:0,5 und 1:1000, beispielsweise zwischen 1:1 und 1:5,00 und insbesondere zwischen, 1:1 und 1:200.

Das 5'-Ende des "Futter"-Oligonukleotids kann unmodifiziert sein oder zusätzlich eine Modifikation tragen. Die 5'-Modifikation ist im wesentlichen unkritisch im Sinne dieser Erfindung, solange dadurch die Bindungseigenschaften des Oligonukleotids zur Template-DNA und die Bindung der High Fidelity DNA-Polymerase nicht negativ beeinflußt werden. Denkbare Modifikationen umfassen C-Linker (e.g. C3-, C6-, C9-, C12-, C18-Linker), 5'-Phosphat-Modifikationen, Amino-Modifikationen , Haptene incl. DIG, Fluorescein, Biotin, Fluoreszenz-Farbstoffe, Quencher-Reste, Thiol-Labels und sonstige allgemein bekannte 5'-Modifikationen, welche die Bindung des Oligonukleotids und der High Fidelity DNA-Polymerase an dem Zielsubstrat nicht behindern.

Interne Modifikation des "Futter"-Oligonukleotids sind ebenfalls möglich und können vorteilhaft im Sinne dieser Erfindung sein. Interne Modifikationen können sowohl die Basen als auch das Zucker-Phosphat-Rückgrat betreffen. Bereits oben wurde auf mögliche Modifikationen des DNA-Rückgrats näher eingegangen. Neben dem erwähnten Austausch von Phosphat gegen Phosphothioat an einer Position, mehreren oder allen Positionen des DNA-Rückgrats sind auch sonstige bekannte Veränderungen des Rückgrats möglich, solange dadurch die Bindung der High Fidelity DNA-Polymerase nicht komplett verhindert wird. Weiterhin ist auch der Einbau von abasischen Sites durch Spacer möglich, oder es können andere Modifikationen an Basen, Rückgrat oder Seitenketten durchgeführt werden. Besonders erwähnt seien hier die Locked Nucleic Acids, die sich durch einen modifizierten Zucker auszeichnen. Auch der Einbau von RNA ist denkbar.

Eine dritte grundlegende Ausführungsform der vorliegenden Erfindung betrifft den Einsatz von jeglicher Art von doppelsträngiger "Futter"-DNA, welche nicht die Primer-Bindungsstellen der zu amplifizierenden Zielsequenz trägt. Die Länge und Basensequenz der "Futter"-DNA sind dabei vorzugsweise so gewählt, daß diese mindestens während des Annealing-Schritts der PCR als DNA-Doppelstrang vorliegt. Die zugesetzte "Futter"-DNA sollte im bevorzugten Fall zusätzlich so formuliert sein, daß sie in keiner unerwünschten Weise an der PCR-Reaktion teilnimmt und keine Nebenprodukte generiert. Denkbare Lösungen sowie bevorzugte grundsätzliche Eigenschaften der zugesetzten "Futter"-DNA werden im Folgenden näher beschrieben.

Die "Futter"-DNA sollte in der bevorzugten Ausführungsform nicht durch die DNA-Polymerase verlängerbar sein, d.h. nicht selbst als Primer, dienen können. Dies kann durch Modifikation des 3'-Endes der Futter-DNA erreicht werden, so daß diese nicht mehr durch die High Fidelity DNA-Polymerase verlängert werden kann. Hierfür gibt es verschiedene Lösungen. Dazu gehört der Einbau von Didesoxy-Nukleotiden, inversen Basen, Spacern, Farbstoffen, Quencher-Resten wie z.B. Black Hole Quencher, Dabcyl, Minor-Groove-Binder, modifizierten Basen wie z.B. Super-Basen oder halogenierte Basen, oder Basen-Analoga, abasischen Sites, die Blockierung der 3'-OH Gruppe (z.B. Ersatz durch Phosphate) sowie alle anderen denkbaren Modifikationen am Zucker-Rückgrat, den Basen sowie zusätzliche Seitengruppen, welche die katalytische Fähigkeit der High Fidelity DNA-Polymerase zur DNA-Synthese inhibieren. Dies kann z.B. auch durch enzymatische oder chemische Modifikation erfolgen. Eine weitere Möglichkeit ist die Verwendung von zirkulären DNA-Molekülen als "Futter"-DNA. Diese zirkuläre DNA kann z.B. eine Plasmid-DNA sein, oder es kann sich um kleinere, synthetisch hergestellte ringförimge DNA-Moleküle handeln.

Bedingt durch ihre 3'-5'-Exonuklease-Aktivität ist die High Fidelity DNA-Polymerase in der Lage, eine "Futter"-DNA am 3'-Ende zu verkürzen, wodurch die Modifikation zur Verhinderung der 3'-Verlängerung unerwünschterweise entfernt werden könnte. Diese 3'-5'-exonukleolytische Verkürzung kann durch Veränderungen im Rückgrat der DNA verhindert werden. Bevorzugte Lösung dafür ist wiederum der Einbau von mindestens einem Phosphothioat anstatt eines Phosphats im Zucker-Phopshat-Rückgrat des DNA-Oligonukleotids. Dies kann z.B. durch enzymatische oder chemische Modifikation erfolgen. Um eine 3'-5'-exonukleolytische Verkürzung komplett zu unterbinden reicht es aus, das Phosphat im Rückgrat zwischen der letzten und vorletzten 3'-Base des DNA-Oligonukleotids durch ein Phosphothioat zu ersetzen. Eine weitere Möglichkeit zur Verhinderung der exonukleolytischen Verkürzung kann durch die Verwendung von Peptide Nucleic Acids (PNA) erreicht werden.

Die "Futter"-DNA sollte gemäß eines weiteren bevorzugten Aspekts aus einer Schar von DNA-Molekülen mit möglichst vielen verschiedenen Sequenzmotiven bestehen. Dadurch wird erreicht, daß in jeglicher verwendeten Template-Nukleinsäure immer ausreichend geeignete Bindungsstellen vorliegen, was vorteilhaft für die generische Verwendbarkeit der ausgewählten "Futter"-DNA ist. Die "Futter"-DNA kann verschiedene Modifikationen sowohl an den DNA-Basen als auch im Rückgrat der DNA tragen. Es können auch universelle oder modifizierte Basen vorhanden sein oder Modifikationen an den Seitenketten eingefügt werden.

Ein wesentlicher Aspekt dieser Erfindung ist, daß, wie oben bereits erwähnt, die als "Futter"-DNA eingesetzte DNA während der PCR-Reaktion in der Lage ist, doppelsträngige Strukturen mit anderen "Futter"-DNA-Molekülen oder der Template-DNA auszubilden. Diese doppelsträngigen Strukturen dienen als Bindungsstelle für die High Fidelity DNA-Polymerase. Die Bindungsstellen können sowohl durch Bindung des "Futter"-Oligonukleotids auf der als Template eingesetzten Nukleinsäure entstehen als auch auf dem als "Futter"-DNA eingesetzten Haarnadel-Oligonukleotid selbst vorliegen.

Die Basensequenz der verwendeten "Futter"-DNA sollte vorzugsweise so gestaltet sein, daß durch die Gegenwart der "Futter"-DNA keine allgemein auftretenden Nebenprodukte in störender Menge entstehen oder die Futter-DNA per se detektierbar ist. Eine störende Menge ist im allgemeinen dann erreicht, wenn die generierten Nebenprodukte bei der verwendeten Analysemethode der PCR-Produkte als Hintergrund nachweisbar werden oder einen störenden Einfluss bei nachgeschalteten Anwendungen, wie z.B. Klonierungen, *in vitro* Transkription/Translation oder Mutagenese, zeigen.

Die Bindungsstellen, an denen ein DNA-Doppelstrang ausgebildet wird, müssen nicht perfekt komplementär sein, sondern es können auch Bindungen mit einer Basen-Fehlpaarung oder mehreren Basen-Fehlpaarungen auftreten. Eine "Futter"-DNA im Sinne der Erfindung sollte vorzugsweise zumindest im Annealing-Schritt der PCR einen DNA-Doppelstrang ausbilden. Das Annealing wird meist in einem Temperaturbereich von ca. 45°C-70°C durchgeführt.

Daraus ergibt sich eine bevorzugte Länge der "Futter"-DNA von ca. 12 bp bis ca. 1000 bp. Die zu wählende bevorzugte Länge hängt von der Sequenz, dem GC-Gehalt und eventuellen sonstigen Modifikationen der "Futter"-DNA ab.

Die "Futter"-DNA sollte vorzugsweise eine Länge aufweisen, bei der unter PCR-Bedingungen die Bildung eines DNA-Doppelstrangs oder die Bindung an einen geeigneten Gegenstrang möglich ist. Das Oligonukleotid sollte demnach mindestens eine Länge aufweisen, die eine effiziente Bindung in einem PCR-Puffer bei ca. 40°C-90°C erlaubt. Typischerweise sollte eine Bindung im Annealing-Schritt der PCR erfolgen, welcher meist in einem Temperaturbereich von ca. 45°C-70°C durchgeführt wird. Daraus ergibt sich eine Länge der "Futter"-DNA von mindestens ca. 12 Basen bis zu mehreren 1000 Basen (beispielsweise ca. 2000, 3000, 4000, 5000 oder 10.000 Basen). Die zu wählende bevorzugte Länge hängt u.a. auch von der Sequenz, dem GC-Gehalt und eventuellen sonstigen Modifikationen der "Futter"-DNA ab.

Die optimale Konzentration wird durch die Länge und Basensequenz sowie die sonstigen Eigenschaften der "Futter"-DNA bestimmt. Die bevorzugte Konzentration liegt für "Futter"-DNA mit einer Länge von ca. 12 bp bis ca. 1000 bp zwischen 0,2 µM und 2,5 µM. Zusätzlich hängt die effektive Konzentration auch von der Konzentration der High Fidelity DNA-Polymerase ab. Geht man dabei davon aus, dass 1 U High Fidelity DNA-Polymerase ungefähr 1 pmol Polymerase-Molekülen entsprechen, dann ergibt sich ein bevorzugtes Verhältnis von High Fidelity DNA-Polymerase-Molekülen zur Futter-DNA von 1 Molekül High Fidelity DNA Polymerase pro 20 bis 1000 Basenpaare zugesetzter "Futter" DNA-Moleküle.

Das 5'-Ende der "Futter"-DNA kann unmodifiziert sein oder zusätzlich eine Modifikation tragen. Die 5'-Modifikation ist im wesentlichen unkritisch im Sinne der Erfindung, solange dadurch die Bindungseigenschaften des Oligonukleotids zur Template-DNA und die Bindung der High Fidelity DNA-Polymerase nicht beeinträchtigt werden. Mögliche Modifikationen umfassen C-Linker (z.B. C3-, C6-, C9-, C12-, C18-Linker), 5'-Phosphat-Modifikationen, Amino-Modifikationen, Haptene incl. DIG, Fluorescein, Biotin, Fluoreszenz-Farbstoffe, Quencher-Reste, Thiol-Labels und sonstige bekannte 5'- Modifikationen, welche die Bindung der der "Futter"-DNA und der High Fidelity DNA-Polymerase nicht verhindern.

Interne Modifikationen der "Futter"-DNA sind ebenfalls möglich und können vorteilhaft im Sinne dieser Erfindung sein. Interne Modifikationen können die Basen oder das Zucker-Phosphat Rückgrat betreffen. Bereits oben wurde näher auf mögliche Modifikationen des DNA-Rückgrats eingegangen. Neben dem erwähnten Austausch von Phosphat gegen Phosphothioat an einer Position oder mehreren oder allen Positionen des DNA-Rückgrats sind alle aus dem Stand der Technik bekannten Veränderungen des Rückgrats möglich, solange dadurch die Bindung der High Fidelity DNA-Polymerase an den DNA-Doppelstrang nicht verhindert wird. Weiterhin ist der Einbau von abasischen Sites durch Spacer oder andere Modifikationen an Basen, Rückgrat oder Seitenketten möglich. Besonders erwähnt seien hier die Locked Nucleic Acids, die sich durch einen modifizierten Zucker auszeichnen. Auch der Einbau von RNA ist denkbar.

Eine vierte bevorzugte Ausführungsform der Erfindung schließlich betrifft den Zusatz von RNA als Zielsubstrat bzw. "Futter" zu einer PCR.

Eine weitere mögliche Lösung im Sinne der Erfindung ist der Zusatz von RNA zur PCR mittels einer High Fidelity DNA-Polymerase. Überraschenderweise zeigt der Zusatz von RNA zur PCR mittels einer High Fidelity DNA-Polymerase den gewünschten positiven Effekt auf die Verbesserung der Sensitivität der PCR im Sinne der Erfindung. Der wahrscheinlichste Mechanismus ist die Ausbildung von doppelsträngigen RNA-DNA-Hybriden, welche durch die High Fidelity DNA-Polymerase erkannt werden können. Denkbar ist auch die Erkennung von RNA-RNA Doppelsträngen durch die High Fidelity DNA-Polymerase. Die Länge und Basensequenz der "Futter"-RNA sind dabei so gewählt, daß diese mindestens während des Annealing-Schritts der PCR in der Lage ist, eine doppelsträngige Struktur auszubilden. Die zugesetzte "Futter"-RNA kann zusätzlich so formuliert sein, daß sie nicht in unerwünschter Weise an der PCR-Reaktion teilnimmt und auch keine Nebenprodukte generiert. Eine solche Modifikation muss aber nicht notwendiger Weise gegeben sein, um eine Verbesserung der PCR-Produktausbeute und Sensitivität im Sinne der Erfindung zu erzielen. Mögliche technische Lösungen sowie grundsätzliche Eigenschaften der zugesetzten "Futter"-RNA werden im Folgenden näher beschrieben.

Es ist nicht zu erwarten, daß die "Futter"-RNA selbst als Primer oder Template für die High Fidelity DNA-Polymerase dienen kann. Modifikationen des 3'-Endes der Futter-RNA sind im Sinne dieser Erfindung daher nicht unbedingt notwendig, können aber durchgeführt werden.

Hierfür sind verschiedene Lösungen geeignet, wobei die Modifikationen vorzugsweise über enzymatische oder chemische Prozesse erzeugt werden. Dazu gehören der Einbau von Didesoxy-Nukleotiden, inversen Basen, Spacern, Farbstoffen, Quencher-Resten wie z.B. Black Hole Quencher, Dabcyl, Minor-Groove-Binder, modifizierten Basen wie z.B. Super-Basen, halogenierte Basen, oder Basen-Analoga, abasischen Sites, Einbau von DNA mit blockierter 3'-OH-Gruppe (z.B. Ersatz der 3'-OH-Gruppe durch Phosphat) sowie alle anderen bekannten Modifikationen am Zucker-Rückgrat, wie z.B. 2-O-Methyl-RNA, und den Basen, sowie zusätzliche Seitengruppen, welche die katalytische Fähigkeit der High Fidelity DNA-Polymerase zur DNA-Synthese inhibieren. Dies kann durch enzymatische oder chemische Modifikation erfolgen. Eine weitere Möglichkeit ist die Verwendung von zirkulären RNA-Moleküle als "Futter"-RNA. Dies können z.B. kleinere, synthetisch hergestellte, ringförmige RNA-Moleküle sein.

Die "Futter"-RNA sollte daher vorzugsweise aus einer Schar von RNA-Molekülen mit möglichst vielen verschiedenen Sequenzmotiven bestehen. Dadurch wird erreicht, daß in jeglicher verwendeten Template-Nukleinsäure immer ausreichend geeignete Bindungsstellen vorliegen, was vorteilhaft für die generische Verwendbarkeit der ausgewählten "Futter"-RNA ist. Es konnte allerdings überraschenderweise auch ein vergleichbarer Effekt mit RNA-HomoPolymeren (Poly-A-RNA, siehe Beispiel 8) erzielt werden. Die "Futter"-RNA kann verschiedene Modifikationen sowohl an den Basen als auch im Zucker-Phosphat-Rückgrat tragen. Es können auch universelle oder modifizierte Basen vorhanden sein oder Modifikationen an den Seitenketten eingefügt werden.

Ein wesentlicher Aspekt dieser Erfindung ist, daß die als "Futter"-RNA eingesetzte RNA während der PCR-Reaktion in der Lage ist, doppelsträngige Strukturen mit sich selbst oder anderen vorhandenen Nukleinsäuremolekülen auszubilden. Die Basensequenz der verwendeten "Futter"-RNA sollte so gestaltet sein, daß durch die Gegenwart der "Futter"-RNA keine allgemein auftretenden Nebenprodukte in störender Menge entstehen oder die Futter-RNA per se detektierbar ist. Eine störende Menge ist im allgemeinen dann erreicht, wenn die generierten Nebenprodukte bei der verwendeten Analysemethode der PCR-Produkte als Hintergrund nachweisbar werden oder einen störenden Einfluss bei nachgeschalteten Anwendungen, wie z.B. Klonierungen, *in vitro* Transkription/Translation oder Mutagenese, zeigen.

Die Bindungsstellen, an denen ein Doppelstrang ausgebildet wird, müssen nicht perfekt komplementär sein, sondern es dürfen auch Bindungen mit einer Basen-Fehlpaarung oder mehreren Basen-Fehlpaarungen auftreten. Eine "Futter"-RNA im Sinne der Erfindung sollte vorzugsweise zumindest im Annealing-Schritt der PCR einen Doppelstrang ausbilden können. Das Annealing wird meist in einem Temperaturbereich von ca. 45°C-70°C durchgeführt. Daraus ergibt sich eine bevorzugte Länge der "Futter"-RNA von mindestens ca. 12 Basen bis zu mehreren 1000 Basen (z.B. 2000, 3000, 4000, 5000 oder 10.000 Basen). Die zu wählende bevorzugte Länge hängt von der Sequenz, dem GC-Gehalt und eventuellen sonstigen Modifikationen der "Futter"-RNA ab.

Die "Futter"-RNA sollte vorzugsweise eine Länge aufweisen, bei der unter PCR Bedingungen die Bildung eines Doppelstrangs oder die Bindung an einen geeigneten Gegenstrang möglich ist. Das Oligonukleotid sollte demnach mindestens eine Länge aufweisen, die eine effiziente Bindung in einem PCR Puffer bei ca. 40°C-90°C erlaubt. Typischerweise sollte eine Bindung im Annealing-Schritt der PCR erfolgen, welcher meist in einem Temperaturbereich von ca. 40°C-70°C durchgeführt wird. Daraus ergibt sich eine bevorzugte Länge der "Futter"-RNA von mindestens ca. 12 Basen bis zu mehreren 1000 Basen (ca. 2000, 3000, 4000, 5000 oder 10.000 Basen). Die zu wählende bevorzugte Länge hängt u.a. von der Sequenz, dem GC-Gehalt und eventuellen sonstigen Modifikationen der "Futter"-RNA ab.

Die effektive Konzentration der "Futter"-RNA liegt vorzugsweise in einem Bereich von ca. 0,04 bis 40 ng/µl. Die optimale Konzentration wird durch die Länge und Basensequenz sowie die sonstigen Eigenschaften der "Futter"-RNA bestimmt. Die bevorzugte Konzentration liegt für eine "Futter"-RNA mit einer Länge von ca. 12 Basen bis ca. 10.000 Basen zwischen ca. 0,04 ng/µl bis 40 ng/µl.

Das 5'-Ende der "Futter"-RNA kann unmodifiziert sein oder zusätzlich eine Modifikation tragen. Die 5'-modifikation ist im wesentlichen unkritisch im Sinne der Erfindung, solange dadurch die Bindungseigenschaften der "Futter"-RNA nicht in der Weise beeinflußt werden, daß die Bindung der High Fidelity DNA-Polymerase verhindert wird. Geeignete Modifikationen umfassen C-Linker (e.g. C3-, C6-, C9-, C12-, C18-Linker), 5'-Phosphat-Modifikationen, Amino-Modifikationen, Haptene incl. DIG, Fluorescein, Biotin, Fluoreszenz-Farbstoffe, Quencher-Reste, Thiol-Labels und sonstige bekannte 5'- Modifikationen, welche die Bindung der High Fidelity DNA-Polymerase nicht verhindern.

Interne Modifikation der "Futter"-RNA sind ebenfalls möglich und können vorteilhaft im Sinne dieser Erfindung sein. Interne Modifikationen können die Basen, das Zucker-Phosphat-Rückgrat oder eingefügte Seitenketten betreffen. Im allgemeinen sind alle bekannten Veränderungen des RNA-Rückgrats denkbar, solange dadurch die Bindung der "Futter"-RNA oder der High Fidelity DNA-Polymerase nicht verhindert wird.

### In den Figuren zeigen

- Fig. 1: eine Agarosegel-Analyse einer PCR zur Untersuchung des Effekts von "Futter"-Oligonukleotiden unterschiedlicher Länge auf die PCR-Amplifikation von ERCC1;
- Fig. 2: eine Agarosegel-Analyse einer PCR zur Untersuchung des Effekts von Haarnadel-"Futter"-Oligonukleotiden auf die PCR-Amplifikation eines 1,5 kb Fragments aus dem humanen CFTR Genlokus;
- Fig. 3: eine Agarosegel-Analyse von PCR-Produkten zur Untersuchung des Effekts von "Futter"-Oligonukleotiden unterschiedlicher Länge auf die PCR-Amplifikation eines 1,5 kb Fragments aus dem humanen CFTR Genlokus;
- Fig. 4A bis 4D: Agarosegel-Analysen von PCR-Produkten zur Untersuchung des Effekts von Oligonukleotiden mit unterschiedlichen Eigenschaften auf die PCR-Amplifikation von β-Actin;
- Fig. 5A bis 5C: Agarosegel-Analysen von PCR-Produkten zur Untersuchung des Effekts von einzelsträngigen 45mer "Futter"-Oligonukleotiden, die sich durch die Anwesenheit eines Phosphothioat im DNA-Rückgrat unterscheiden, auf die PCR-Amplifikation von β-Actin;
- Fig. 6A bis 6C: Agarosegel-Analysen zur Untersuchung des Effekts von "Futter"-Oligonukleotiden, die abasische Spacer enthalten, auf die PCR Amplifikation von β-Actin;
- Fig. 7A und 7B: Agarosegel-Analysen zur Untersuchung des Effekts von RNA, als "Futter" im' Sinne der Erfindung auf die PCR-Amplifikation von β-Actin;
- Fig. 8A und 8B: Agarosegel-Analysen zur Untersuchung des Effekts des Zusatzes von Poly-A-RNA auf die PCR Amplifikation von β-Actin;
- Fig. 9: eine Agarosegel-Analyse zur Untersuchung des Effekts von PCR-Primern, die zwischen der letzten und vorletzten 3'-Base ein Phosphothioat im Zucker-Phosphat-Rückgrat der DNA tragen, auf die Amplifikation von β-Actin im Vergleich zu Standard PCR-Primern;
- Fig. 10: eine Agarosegel Analyse zur Untersuchung des Effekts von Haarnadel-"Futter"-Oligonukleotiden auf die PCR-Amplifikation eines 2 kb Fragments aus dem murinen PKC Genlokus unter Verwendung des Enzyms VentR;
- Fig. 11: eine Agarosegel-Analyse zur Untersuchung des Effekts von "Futter"-Oligonukleotiden auf die PCR-Amplifikation von β-Actin unter Verwendung des Enzyms VentR;
- Fig. 12: eine Agarosegel-Analyse zur Untersuchung des Effekts von "Futter"-Oligonukleotiden auf die PCR-Amplifikation eines 2 kb Fragments aus dem murinen PKC Genlokus unter Verwendung des Enzyms VentR; und
- Fig. 13: eine Agarosegel-Analyse zur Untersuchung des Effekts des "Futter"-Oligonukleotids N40-Thio in Verbindung mit variablen Konzentrationen der PCR-Primer auf die Amplifikation von β-Actin.

Nachfolgend wird die Erfindung anhand von Ausführungsbeispielen näher erläutert.

### Beispiel 1

In diesem Beispiel wird der Effekt von "Futter"-Oligonukleotiden unterschiedlicher Länge (P20-GAPDH; P30-GAPDH; P45-GAPDH und Random-Octamer) auf die PCR-Amplifikation von ERCC1 mittels High Fidelity DNA-Polymerase untersucht. Die Oligonukleotide P20-GAPDH (20-mer); P30-GAPDH (30-mer); und P45-GAPDH (45-mer) sind komplementär zum humanen GAPDH Genlokus. Es handelt sich um einzelsträngige Oligonukleotide mit natürlichem Zucker-Phosphat-Rückgrat, die anstatt der endständigen 3'-OH-Gruppe ein 3'-Phosphat tragen. Der Random-Octamer ist ein 8-mer mit zufälliger Sequenz (N₈). Es handelt sich ebenfalls um ein einzelsträngiges Oligonukleotid mit natürlichem Zucker-Phosphat-Rückgrat und einer 3'-OH-Gruppe.

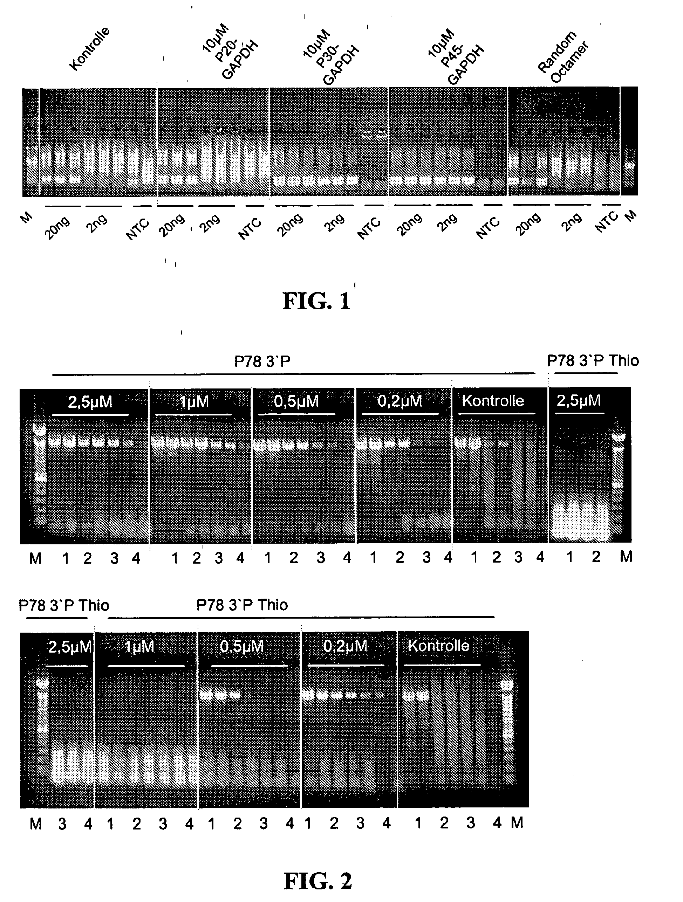

Der Ansatz und die Durchführung der High Fidelity PCR waren wie folgt. Für die PCR-Reaktionen wurde ProofStart DNA-Polymerase verwendet (QIAGEN GmbH, Hilden Deutschland; Katalog Nummer 202205). Jeder Reaktionsansatz enthielt 1x ProofStart PCR-Buffer, 1,25 U ProofStart DNA-Polymerase, je 1 µM ERCC1-Vorwärts- und Rückwärts-Primer und je 0,3 mM dNTPs (dATP, dCTP, dGTP, dTTP) in einem Reaktionsvolumen von 25 µl. Den Dreifachansätzen (d.h. drei Parallelversuche) wurde jeweils 20 ng oder 2 ng humane genomische DNA und in Zweifachansätzen (d.h. zwei Parallelversuche) eine vergleichbare Menge Wasser als Negativkontrolle (NTC: "No-Templäte-Control") zugesetzt. Zusätzlich wurden als "Futter"-Oligonukleotid im Sinne der Erfindung jeweils eines der Oligonukleotide P20-GAPDH; P30-GAPDH; P45-GAPDH sowie ein "Random Octamer" in einer Konzentration von 10 µM zugesetzt. Als Kontrolle wurden jeweils entsprechende Reaktionen ohne "Futter"-Oligonukleotid mitgeführt (Beschriftung: "Kontrolle" in Fig. 1).

Das PCR-Protokoll bestand aus einer initialen Reaktivierung der ProofStart DNA-Polymerase ("Hot-Start") für 5 min bei 95°C, gefolgt von 35 Zyklen mit 30 s bei 94°C, 60 s bei 61°C und 1 min 30 s bei 72°C sowie einem abschließenden Schritt für 10 min bei 72°C ("final extension"). Die nachfolgenden Sequenzen von Oligonukleotiden und Primern sind in 5'-3'-Richtung angegeben.

### PCR-Primer Sequenzen:

ERCC1-for: GCT GTT TGA TGT CCT GCA CGA G
ERCC1-rev: GCC TGG CCT GGG AGG ACG ATT

### Sequenzen der "Futter"-Oligonukleotide:

P20-GAPDH: GCG TCA AAG GTG GAG GAG TG [Phosp-Q]
P30-GAPDH: GCG TCA AAG GTG GAG GAG TGG GTG TCG CTG [Phosp-Q]
P45-GAPDH: GCG TCA AAG GTG GAG GAG TGG GTG TCG CTG TTG AAG TCA GAG GAG [Phosp-Q]
   ([Phosp-Q]: 3'-Phosphat anstelle von 3'-OH)
   Random Octamer: NNN NNN NN

10 µl jeder PCR Reaktion wurden auf einem mit Ethidiumbromid gefärbten Agarosegel (2 %) analysiert. Als Größenstandard diente eine 100 bp Leiter (Invitrogen GmbH, Karlsruhe, Deutschland, 15628-050). Fig. 1 zeigt eine Agarosegel-Analyse der PCR hinsichtlich des Effekts von "Futter" Oligonukleotiden unterschiedlicher Länge (P20-GAPDH; P30-GAPDH; P45-GAPDH und Random Octamer) auf die PCR-Amplifikation von ERCC1. M=Marker.

Aus Fig. 1 läßt sich entnehmen, daß eine Verbesserung der Ausbeute im Vergleich zur Kontrolle bei Verwendung von 20 ng Template-DNA war bei allen Bedingungen zu beobachten ist. Eine erfolgreiche Amplifikation, d.h. eine Verbesserung der Sensitivität um Faktor 10, wurde bei Verwendung der "Futter"-Oligonukleotide P30-GAPDH (30mer) und P45-GAPDH (45mer) erzielt. Gleichzeitig wurde auch der bei High-Fidelity PCR-Reaktionen häufig als "Schmier" auftretende Hintergrund deutlich reduziert.

Dies legt den Schluß nahe, daß Oligonukleotide, die aufgrund ihres Schmelzpunkts (bedingt durch Länge und GC-Gehalt) in der Lage sind unter PCR-Bedingungen einen DNA Doppelstrang auszubilden, besonders als "Futter-Oligonukleotid" im Sinne der Erfindung geeignet sind.

### Beispiel 2

In diesem Beispiel wurde der Effekt der Haarnadel "Futter"-DNA-Oligonukleotide P78 3'P und P78 3'p-Thio auf die PCR mit High Fidelity DNA-Polymerase untersucht. Die Oligonukleotide enthalten in ihrem 5'-Bereich eine Sequenz, die komplementär zum humanen GAPDH Genlokus ist. Der 3'-Bereich ist komplementär zum 5'-Bereich des Oligonukleotids, so daß eine doppelsträngige Haarnadelstruktur ausgebildet werden kann. Es handelt sich um Oligonukleotide, die anstelle der 3'-OH-Gruppe ein 3'-Phosphat tragen. Das Oligonukleotid P78 3'P-Thio trägt zwischen der letzten und der vorletzten 3'-Base ein Phosphothioat im Zucker-Phosphat-Rückgrat der DNA.

Der Ansatz und die Durchführung der PCR mittels High Fidelity DNA-Polymerase waren wie folgt. Für die PCR-Reaktionen wurde ProofStart DNA-Polymerase verwendet (QIAGEN, Katalog Nummer 202205). Jeder Reaktionsansatz enthielt 1x ProofStart PCR-Buffer, 1,25 U ProofStart DNA-Polymerase, je 1 µM Vorwärts- und -Rückwärts-Primer für CFTR (CYST) und je 0,3 mM dNTPs (dATP, dCTP, dGTP, dTTP) in einem Reaktionsvolumen von 25 µl. Den Zweifachansätzen wurde jeweils 25 ng, 5 ng oder 1 ng humane genomische DNA und in Einzelbestimmung eine vergleichbare Menge Wasser als Negativkontrolle zugesetzt.(NTC: "No-Template-Control"). Zusätzlich wurden als "Futter"-Oligonukleotid im Sinne der Erfindung jeweils eines der Oligonukleotide P78 3'P und P78 3'p-Thio in einer Konzentration von 2,5 µM, 1 µM, 0,5 µM und 0,2 µM zugesetzt. Als Kontrolle wurden jeweils entsprechende Reaktionen ohne "Futter" Oligonukleotid mitgeführt (Beschriftung in Fig. 2: "Kontrolle"). Während der Vorbereitung der PCR Reaktionen wurde die Proben gekühlt.

Das PCR-Protokoll umfaßte eine initiale Reaktivierung der ProofStart DNA-Polymerase ("Hot-Start") für 5 min bei 95°C, gefolgt von 35 Zyklen mit 30 s bei 94°C, 60 s bei 61°C und 1 min 30 s bei 72°C, sowie einem abschließenden Schritt für 10 min bei 72°C ("final extension"). Die nachfolgenden Sequenzen von Oligonukleotiden und Primern sind in 5'-3' Richtung angegeben.

### PCR-Primer Sequenzen:

CYST 3: CCC AAA CCC AAC CCA TAC ACA C
CYST 5: CCT TGC CTT AGA TGT GTC GGC A

### Sequenzen der "Futter"-Oligonukleotide:

P78 3′P: GCG TCA AAG GTG GAG GAG TGG GTG TCG CTG TTG AAG TCA TGA
CTT CAA CAG CGA CAC CCA CTC CTC CAC CTT TGA CGC [Phosp-Q] ([Phosp-Q]:

3'-Phosphat anstelle von 3'-OH)
P78 3'p-Thio: GCG TCA AAG GTG GAG GAG TGG GTG TCG CTG TTG AAG TCA TGA CTT CAA CAG CGA CAC CCA CTC CTC CAC CTT TGA CG *C[Phosp-Q] ([Phosp-Q]: 3'-Phosphat anstelle von 3'-OH; *: Phosphothioat im Rückgrat)

10 µl jeder PCR Reaktion wurden auf einem mit Ethidiumbromid gefärbten Agarosegel (1 %) analysiert. Als Größenstandard diente eine 100 bp Leiter (Invitrogen, 15628-050).

Fig. 2 zeigt die Agarosegel-Analyse der PCR zur Untersuchung des Effekts von Haarnadel-"Futter"-Oligonukleotiden (P78 3'P, P78 3'P-Thio) auf die PCR-Amplifikation eines 1,5 kb Fragments aus dem humanen CFTR Genlokus. Spur 1: 25 ng HuDNA; Spur 2: 5 ng HuDNA; Spur 3: 1ng HuDNA; Spur 4: NTC; M=Marker.

Aus Fig. 2 ist ersichtlich, daß durch Zusatz der Haarnadel "Futter"-DNA-Oligonukleotide P78 3'P und P78 3'p-Thio die Ausbeute und Sensitivität der High-Fidelity PCR des 1,5 kb Fragments deutlich im Vergleich zur Kontrolle ohne "Futter"-DNA-Oligonukleotide erhöht werden konnte. Gleichzeitig wurde auch der bei High-Fidelity PCR-Reaktionen häufig als "Schmier" auftretende Hintergrund deutlich reduziert.

Das "Futter"-DNA-Oligonukleotid P78 3'P hatte im gesamten getesteten Bereich von 0,2 µM bis 2,5 µM einen positiven Effekt auf Ausbeute und Sensitivität der High-Fidelity PCR Amplifikation des 1,5 kb Fragments.

Das "Futter"-DNA-Oligonukleotid P78 3'p-Thio zeigte bei der Amplifikation des 1,5 kb Fragments einen positiven Effekt im Bereich von 0,2 µM bis 0,5 µM. Bei höheren Konzentrationen war nur noch das Haarnadel "Futter"-DNA-Oligonukleotid auf dem Gel als Schmier um 200 bp nachweisbar. Dieser Effekt tritt nicht bei dem "Futter"-DNA-Oligonukleotid P78 3'P auf.

Dies legt den Schluß nahe, daß das "Futter"-DNA-Oligonukleotid P78 3'P durch die 3'-5'-Exonuklease-Aktivität der High-Fidelity DNA Polymerase abgebaut wird. Dadurch ist es möglich, wesentlich höhere Konzentrationen "Futter"-DNA-Oligonukleotid P78 3'P im Vergleich zu P78 3'p-Thio einzusetzen. Durch Kombination von 3'-Phosphat mit einem Phosphothioat, wie bei P78 3'p-Thio, bleibt das "Futter"-DNA-Oligonukleotid offensichtlich intakt und ist somit in der Lage, während der gesamten PCR die High-Fidelity DNA-Polymerase zu binden.

### Beispiel 3

In diesem Beispiel wurde der Effekt von "Futter"-Oligonukleotiden, die einen randomisierten Teil oder die universelle Base Inosin enthalten (N14-degAATAAA; pA-I; βActpA-61-3'), auf die PCR-Amplifikation eines 1,5 kb Fragments aus dem humanen CFTR Genlokus untersucht.

Die Oligonukleotide N14-degAATAAA, pA-1, und βActpA-6I-3' (Sequenz siehe unten) werden auf ihre Leistungsfähigkeit in der PCR getestet. Es handelt sich um einzelsträngige Oligonukleotide mit natürlichem Zucker-Phosphat Rückgrat, die eine endständige 3'-OH Gruppe tragen.

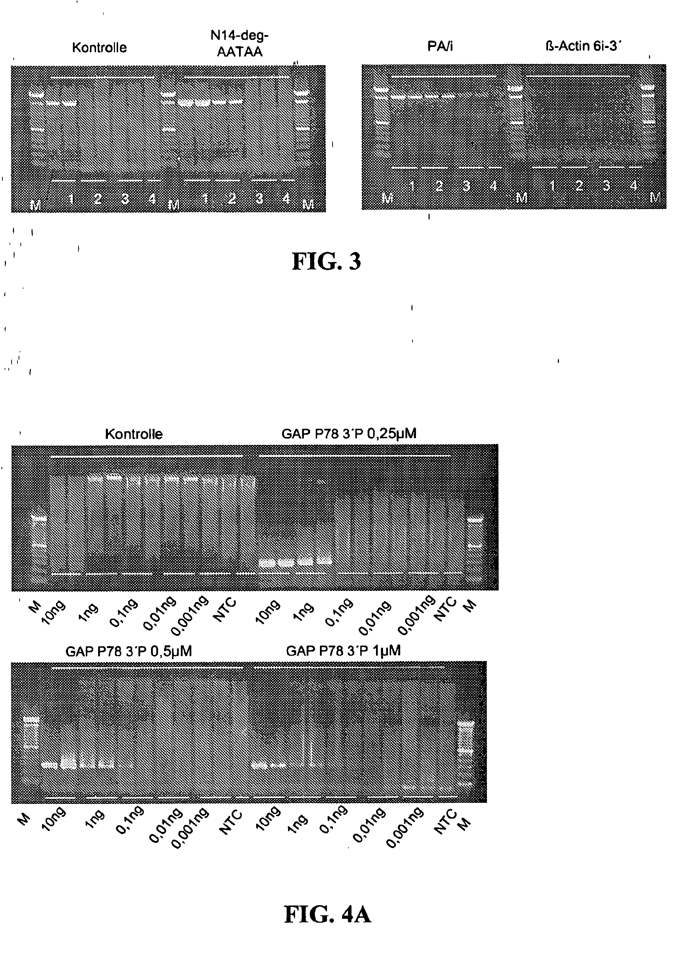

Der Ansatz und die Durchführung der PCR mittels DNA-Polymerase waren wie folgt. Für die PCR-Reaktionen wurde ProofStart DNA-Polymerase verwendet (QIAGEN, Katalog Nummer 202205). Jeder Reaktionsansatz enthielt 1x ProofStart PCR-Buffer, 1,25 U ProofStart DNA-Polymerase, je 1 µM CYST Vorwärts- und -Rückwärts-Primer und je 0,3 mM dNTPs (dATP, dCTP, dGTP, dTTP) in einem Reaktionsvolumen von 25 µl Den Zweifach-Ansätzen wurden jeweils 25 ng, 5 ng oder 1 ng humane genomische DNA zugesetzt, und in einem Einfachansatz wurde eine vergleichbare Menge Wasser als Negativkontrolle (NTC: "No-Template-Control") verwendet. Zusätzlich wurden als "Futter"-Oligonukleotid im Sinne der Erfindung jeweils eines der Oligonukleotide N14-degAATAAA, pA-I und βActpA-6I-3' in einer Konzentration von 10 µM zugesetzt. Als Kontrolle wurden jeweils entsprechende Reaktionen ohne "Futter"-Oligonukleotid mitgeführt (Beschriftung in Fig. 3: "Kontrolle"). Während der Vorbereitung der PCR Reaktionen wurde die Proben gekühlt.

Das PCR Protokoll bestand aus einer initialen Reaktivierung der ProofStart DNA-Polymerase ("Hot-Start") für 5 min bei 95°C, gefolgt von 35 Zyklen mit 30 s bei 94°C, 60 s bei 61°C und 1 min 30 s bei 72°C, sowie einem abschließenden Schritt für 10 min bei 72°C ("final extension"). Die nachfolgenden Sequenzen von Oligonukleotiden und Primern sind in 5'-3' Richtung angegeben.

### PCR Primer-Sequenzen:

CYST3:CCC AAA CCC AAC CCA TAC ACA C ,
CYST 5: CCT TGC CTT AGA TGT GTC GGC A

### Sequenzen der "Futter" Oligonukleotide:

| | |
|---|---|
| N 14-degAATAAA: | NNN NNN NNN NNN NNH HND DVA ATA AA |
| pA-1: | III III III III III III III AAT AAA |
| βActpA-61-3': | GTA CAC TGA CTT GAG ACC AGT TGA ATA AAI III II |

10 µl jeder PCR Reaktion wurden auf einem mit Ethidiumbromid gefärbten Agarosegel (2 %) analysiert. Als Größenstandard diente eine 100 bp Leiter (Invitrogen, 15628-050).

Fig. 3 zeigt eine Agarosegel-Analyse der PCR-Produkte zum Effekt von "Futter"-Oligonukleotiden unterschiedlicher Länge (N14-degAATAAA; pA-I; βAetpA-6I-3') auf die PCR-Amplifikation eines 1,5 kb Fragments aus dem humanen CFTR Genlokus. Spur 1: 25 ng HuDNA; Spur 2: 5 ng HuDNA; Spur 3: 1 ng HuDNA; Spur 4: NTC; M: Marker.

Durch Zusatz der "Futter"-DNA-Oligonukleotide N14-degAATAAA und pA-I konnte die Ausbeute und Sensitivität der High-Fidelity PCR deutlich im Vergleich zur Kontrolle ohne "Futter"-DNA-Oligonukleotide erhöht werden. Gleichzeitig wurde auch der bei High-Fidelity PCR Reaktionen häufig als "Schmier" auftretende Hintergrund deutlich reduziert.

Das "Futter"-DNA-Oligonukleotid βActpA-6I-3' mit 6 Inosin-Basen am 3'-Ende eignete sich nicht als "Futter"-DNA-Oligonukleotid.

Dies legt den Schluß nahe, daß Oligonukleotide mit randomisierten Sequenzen oder universelle Basen wirksam als "Futter"-DNA-Oligonukleotide eingesetzt werden können. Auch eine Verwendung von Standard-Oligonukleotiden ohne Schutz vor Abbau durch die 3'-5'-Exonuklease-Aktivität der verwendeten Polymerase als "Futter"-DNA-Oligonukleotide ist möglich.

### Beispiel 4

In diesem Beispiel wurde der Effekt von "Futter"-DNA-Oligonukleotiden mit unterschiedlichen Eigenschaften auf die PCR mit High Fidelity DNA-Polymerase untersucht. Es wurden die Oligonukleotide GAP P78 3'P, GAP P78 3'p-Thio, N14-degAATAAA und PA-I mit den unten angegebenen Sequenzen als Zusatz in der PCR verwendet. Die Oligonukleotide GAP P78 3'P und GAP P78 3'p-Thio wurden bereits in Beispiel 2 und die Oligonukleotide N14-degAATAAA und PA-I wurden bereits in Beispiel 3 beschrieben.

Der Ansatz und die Durchführung der High Fidelity-PCR waren wie folgt. Für die PCR-Reaktionen wurde ProofStart DNA-Polymerase verwendet (QIAGEN, Katalog Nummer 202205). Jeder Reaktionsansatz enthielt 1x ProofStart PCR-Buffer, 1 U ProofStart DNA-Polymerase, je 1 µM β-Actin Vorwärts- und -Rückwärts-Primer und je 0,3 mM dNTPs (dATP, dCTP, dGTP, dTTP) in einem Reaktionsvolumen von 20 µl.

Den Zweifachansätzen wurden jeweils 10 ng, 1 ng, 0,1 ng, 0,01 ng bzw. 0,001 ng K562 cDNA (aus K562 Zellen, siehe allgemeine Methoden am Ende der Beispiele) und ebenfalls in Zweifachansätzen eine vergleichbare Menge Wasser als Negativkontrolle zugegeben (NTC: "No-Template-Control"). Zusätzlich wurden als "Futter"-Oligonukleotide GAP P78 3'P, GAP P78 3'p-Thio, N14-degAATAAA bzw. PA-I im Sinne der Erfindung jeweils in einer Konzentration von 0,25 µM, 0,5 µM und 1,0 µM zugesetzt. Als Kontrolle wurden jeweils entsprechende Reaktionen ohne "Futter"-Oligonukleotid mitgeführt (Beschriftung in Fig. 4A bis 4D: Kontrolle). Während der Vorbereitung der PCR Reaktionen wurde die Proben gekühlt.

Das PCR Protokoll bestand aus einer initialen Reaktivierung der ProofStart DNA-Polymerase ("Hot-Start") für 5 min bei 95°C, gefolgt von 35 Zyklen mit 30 s bei 94°C, 60 s bei 61°C und 1 min 30 s bei 72°C sowie einem abschließenden Schritt für 10 min bei 72°C ("final extension"). Die nachfolgenden Sequenzen von Oligonukleotiden und Primern sind in 5'-3'-Richtung angegeben.

### PCR Primer-Sequenzen:

BACT-TM.5: TCA CCC ACA CTG TGC CCA TCT ACG A
BACT-TM.3: CAG CGG AAC CGC TCA TTG CCA ATG G

### Sequenzen der "Futter"-Oligonukleotide:

GAP P78 3'P: GCG TCA AAG'G'TG GAG GAG TGG GTG TCG CTG TTG AAG TCA
TGA CTT CAA CAG CGA CAC CCA CTC CTC CAC CTT TGA CGC [Phosp-Q] ([Phosp-Q]: 3'-Phosphat)
GAP P78 3'p-Thio: GCG TCA AAG GTG GAG GAG TGG GTG TCG CTG TTG AAG TCA TGA CTT CAA CAG CGA CAC CCA CTC CTC CAC CTT TGA CG*C [Phosp-Q] ([Phosp-Q]: 3'-Phosphat; *: Phosphothioat im Rückgrat)
N14-degAATAAA: NNN NNN NNN NNN NNH HND DVA ATA AA pA-I: III III III III III III III AAT AAA

5 µl jeder PCR-Reaktion wurden auf einem mit Ethidiumbromid gefärbten Agarosegel (2 %) analysiert. Als Größenstandard diente eine 100 bp Leiter (Invitrogen, 15628-050). Die Fig. 4A bis 4D zeigen Agarosegel-Analysen der PCR-Produkte zum Effekt von Oligonukleotiden mit unterschiedlichen Eigenschaften (GAP P78 'P,GAP P78 3'p-Thio, N14-degAATAAA, PA-I) auf die PCR Amplifikation von β-Actin. M = Marker.

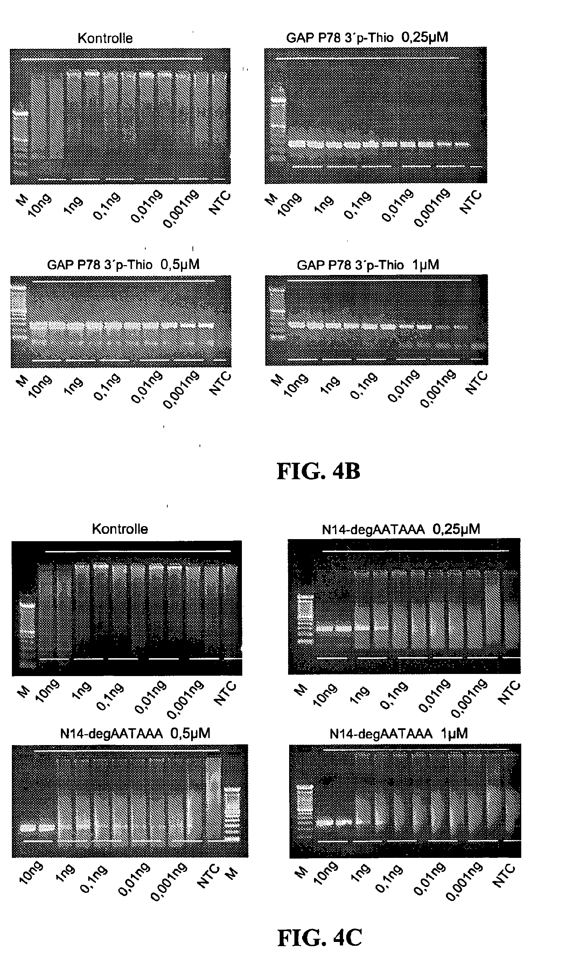

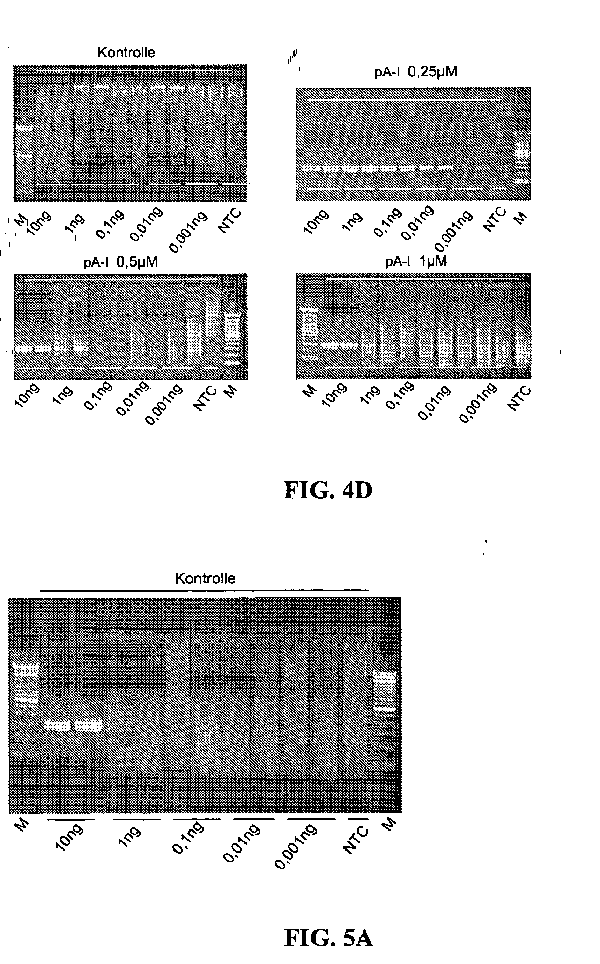

Die bereits erfolgreich in den Beispielen 2 und 3 zur Verwendung mit genomischer DNA getesteten "Futter"-Oligonukleotide wurden in Beispiel 4 mit einer High-Fidelity PCR zur Amplifikation eines 300 bp Fragments aus cDNA als Template-Nukleinsäure eingesetzt. Es konnte in allen Fällen eine deutliche Verbesserung der Ausbeute und Sensitivität erzielt werden. Im Vergleich zu PCR-Reaktionen ohne "Futter"-Oligonukleotid war eine erfolgreiche PCR bei Verwendung von sehr viel geringeren Mengen Template-cDNA möglich Die Sensitivität konnte bis zu 100-fach (Versuch gemäß Fig. 4A), bis zu 10.000-fach (Versuch gemäß Fig. 4B), bis zu 1.000-fach (Versuch gemäß Fig. 4C) und bis zu 10.000-fach (Versuch gemäß Fig. 4D) gesteigert werden. Alle in Beispiel 4 eingesetzten Oligonukleotide eignen sich demnach als "Futter"-Oligonukleotide zur Erhöhung der Ausbeute und Sensitivität einer High-Fidelity PCR.

### Beispiel 5

In diesem Beispiel wurde der Effekt von einzelsträngigen 45mer "Futter"-Oligonukleotiden, die sich durch die Anwesenheit eines Phosphothioats im DNA Rückgrat unterscheiden, auf die PCR Amplifikation von β-Actin mittels High Fidelity DNA-Polymerase untersucht.

Es wurden die Oligonukleotide GAP45-3'P und GAP45-3'P-Thio mit den unten angegebenen Sequenzen als Zusatz in der PCR Verwendet. Es handelt sich um einzelsträngige Oligonukleotide, die anstatt der 3'-OH Gruppe ein 3'-Phosphat tragen. Die beiden Varianten unterscheiden sich durch die Gegenwart (GAP45-3'P-Thio) oder die Abwesenheit (GAP45-3'P) eines Phosphothioats im DNA-Rückgrat.

Der Ansatz und die Durchführung der High Fidelity-PCR waren wie folgt. Für die PCR Reaktionen wurde ProofStart DNA-Polymerase verwendet (QIAGEN, Katalog Nummer 202205). Jeder Reaktionsansatz enthielt 1x ProofStart PCR-Buffer, 1 U ProofStart DNA-Polymerase, je 1 µM β-Actin-Vorwärts- und -Rückwärts-Primer und je 0,3 mM dNTPs (dATP, dCTP, dGTP, dTTP) in einem Reaktionsvolumen von 20 µl. Den Zweifachansätzen wurden jeweils 10 ng, 1 ng, 0,1 ng, 0,01 ng bzw. 0,001 ng K562 cDNA (aus K562 Zellen, siehe allgemeine Methoden am Ende der Beispiele) und ebenfalls in Zweifachansätzen eine vergleichbare Menge Wasser als Negativkontrolle (NTC: "No-Template-Control") zugesetzt. Zusätzlich wurden als "Futter"-Oligonukleotid GAP45-3'P und GAP45-Thio im Sinne der Erfindung jeweils in einer Konzentration von 0,3 µM, 1,0 µM, 3,0 µM bzw. 10 µM zugesetzt. Als Kontrolle wurden jeweils entsprechende Reaktionen ohne "Futter"-Oligonukleotid mitgeführt (Beschriftung in Fig. 5A: "Kontrolle"). Während der Vorbereitung der PCR-Reaktionen wurden die Proben gekühlt.

Das PCR-Protokoll bestand aus einer initialen Reaktivierung der ProofStart DNA-Polymerase ("Hot-Start") für 5 min bei 95°C, gefolgt von 35 Zyklen mit 30 s bei 94°C, 60 s bei 61°C und 1 min 30 s bei 72°C sowie einem abschließenden Schritt für 10 min bei 72°C ("final extension"). Die nachfolgenden Sequenzen von Oligonukleotiden und Primern sind in 5'-3' Richtung angegeben.

### PCR Primer-Sequenzen:

BACT-TM.5: TCA CCC ACA CTG TGC CCA TCT ACG A
BACT-TM.3: CAG CGG AAC CGC TCA TTG CCA ATG G

### Sequenzen der "Futter"-Olieonukleotide:

GAP45-3'P: GCG TCA AAG GTG GAG GAG TGG GTG TCG CTG TTG AAG TCA GAG GAG
GAP45-Thio: GCG TCA AAG GTG GAG GAG TGG GTG TCG CTG TTG AAG TCA GAG GA*G [Phosp-Q] ([Phosp-Q]: 3'-Phosphat; *: Phosphothioat im Rückgrat)

5 µl jeder PCR-Reaktion wurden auf einem mit Ethidiumbromid gefärbten Agarosegel (2 %) analysiert. Als Größenstandard diente eine 100 bp Leiter (Invitrogen, 15628-050). Die Fig. 5A bis 5C zeigen Agarosegel Analysen der PCR-Produkte zur Untersuchung des Effekts von einzelsträngigen 45mer "Futter"-Oligonukleotiden, die sich durch die Anwesenheit eines Phosphothioats im DNA Rückgrat unterscheiden, auf die PCR Amplifikation von β-Actin. M=Marker.

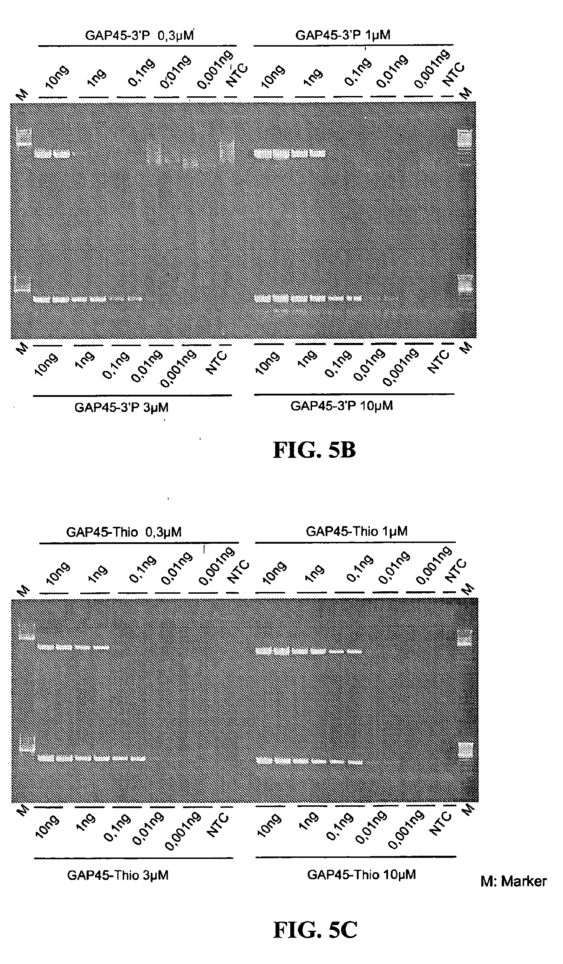

Bei Verwendung der beiden 45mer "Futter"-Oligonukleotide konnte in allen Fällen eine deutliche Verbesserung der Ausbeute und Sensitivität erzielt werden. Im Vergleich zu PCR-Reaktionen ohne "Futter"-Oligonukleotide war eine erfolgreiche PCR bei Verwendung von sehr viel geringeren Mengen Template-cDNA möglich. Die Sensitivität im Vergleich zur Kontrolle (Fig. 5A) konnte bei Verwendung von GAP45-3'P (Fig. 5B) bis zu 1.000-fach und bei Verwendung von GAP45-Thio (Fig. 5C) bis zu 10.000-fach gesteigert werden. Gleichzeitig wurde auch der bei High-Fidelity PCR Reaktionen häufig als "Schmier" auftretende Hintergrund deutlich reduziert.

### Beispiel 6

In diesem Beispiel wurde der Effekt der "Futter" DNA-Oligonukleotide G45-TP-1sp-d und G45-TP-2sp-d, die abasische Spacer enthalten, auf die High Fidelity PCR von β-Actin untersucht. Dazu wurden die Oligonukleotide G45-TP-1sp-d und G45-TP-2sp-d mit den unten angegebenen Sequenzen als Zusatz in der PCR verwendet. Es handelt sich um einzelsträngige Oligonukleotide, die anstatt der 3'-OH Gruppe ein 3'-Phosphat, sowie zwischen der letzten und vorletzten 3'-Base ein Phosphothioat anstelle eines Phosphats im Zucker-Phosphat-Rückgrat der DNA tragen. Die beiden Varianten unterscheiden sich durch die Gegenwart von einem (G45-TP-1sp-d) bzw. zwei (G45-TP-2sp-d) abasischen Spacern.

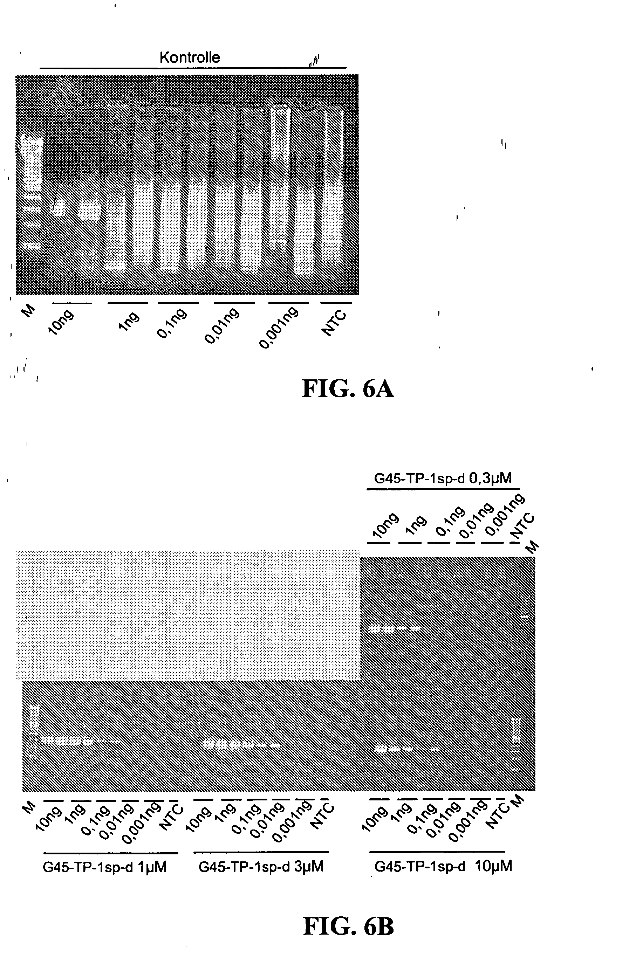

Der Ansatz und die Durchführung der High Fidelity-PCR waren wie folgt. Für die PCR-Reaktionen wurde ProofStart DNA-Polymerase verwendet (QIAGEN, Katalog Nummer 202205). Jeder Reaktionsansatz enthielt 1x ProofStart PCR-Buffer, 1 U ProofStart DNA-Polymerase, je 1 µM β-Actin Vorwärts- und Rückwärts-Primer und je 0,3 mM dNTPs (dATP, dCTP, dGTP, dTTP) in einem Reaktionsvolumen von 20 µl. Den Zweifachansätzen wurden jeweils 10 ng, 1 ng, 0,1 ng, 0,01 ng bzw. 0,001 ng cDNA (aus K562 Zellen, siehe allgemeine Methoden am Ende der Beispiele) und in einem Einfachansatz eine vergleichbare Menge Wasser als Negativkontrolle (NTC: "No-Template-Control") zugesetzt. Zusätzlich wurden als "Futter"-Oligonukleotide im Sinne der Erfindung jeweils eines der Oligonukleotide G45-TP-1sp-d bzw. G45-TP-2sp-d in einer Konzentration von 0,3 µM, 1µM, 3µM und 10µM zugesetzt. Als Kontrolle wurden jeweils entsprechende Reaktionen ohne "Futter"-Oligonukleotid mitgeführt (Beschriftung in Fig. 6A: "Kontrolle"). Während der Vorbereitung der PCR-Reaktionen wurden die Proben gekühlt.

Das PCR Protokoll bestand aus einer initialen Reaktivierung der ProofStart DNA-Polymerase ("Hot-Start") für 5 min bei 95°C, gefolgt von 35 Zyklen mit 30 s bei 94°C, 60 s bei 61°C und 1 min 30 s bei 72°C sowie einem abschließenden Schritt für 10 min bei 72°C ("final extension"). Die nachfolgenden Sequenzen von Oligonukleotiden und Primern sind in 5'-3' Richtung angegeben.

### PCR Primer-Sequenzen:

BACT-TM.5: TCA CCC ACA CTG TGC CCA TCT ACG A
BACT-TM.3: CAG CGG AAC CGC TCA TTG CCA ATG G

### Sequenzen der "Futter" Oligonukleotide:

G45-TP-1sp-d: GCG TCA AAG GTG GAG GAG TGG GT [sp-d]GTC GCT GTT GAA GTC AGA GGA *G[Phosp-Q] ([sp-d]: abasischer spacer [Phosp-Q]: 3' Phosphat)

G45-TP-2sp-d: GCG TCA AAG GT[sp-d] GGA GGA GTG GGT [sp-d]GTC GCT GTT GAA GTC AGA GGA *G[Phosp-Q] ([sp-d]: abasic spacer, [Phosp-Q]: 3' Phosphate; *: Phosphothioat im Rückgrat)

5 µl jeder PCR Reaktion wurden auf einem mit Ethidiumbromid gefärbten 2 % Agarosegel analysiert. Als Größenstandard diente eine 100 bp Leiter (Invitrogen, 15628-050). Die Fig. 6A bis 6C zeigen Agarosegel-Analysen zur Untersuchung des Effekts von "Futter"-Oligonukleotiden, die abasische Spacer enthalten, auf die PCR Amplifikation von β-Actin. M = Marker.

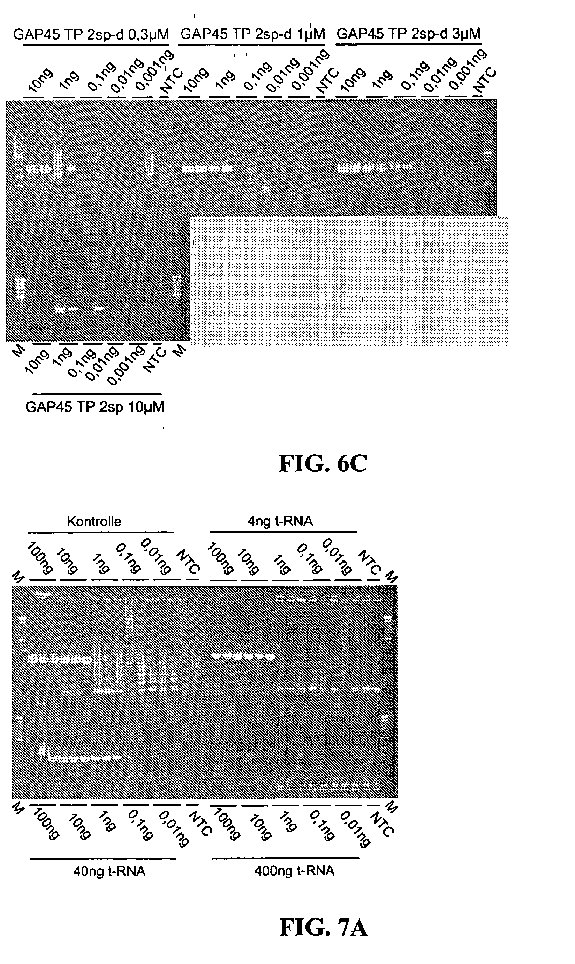

Bei Verwendung der beiden 45mer "Futter"-Oligonukleotide mit einem abasischen Spacer (Fig. 6B) oder zwei abasischen Spacern (Fig. 6C) konnte in allen Fällen eine deutliche Verbesserung der Ausbeute und Sensitivität erzielt werden. Im Vergleich zu PCR-Reaktionen ohne "Futter"-Oligonukleotide (Fig. 6A) war eine erfolgreiche PCR bei Verwendung von sehr viel geringeren Mengen Template-cDNA möglich. Die Sensitivität im Vergleich zur Kontrolle (Fig. 6A) konnte bis zu 1.000-fach (Versuch gemäß Fig. 6B) und bei dem Versuch gemäß Fig. 6C ebenfalls bis zu 1.000-fach gesteigert werden. Gleichzeitig wurde auch der bei High-Fidelity PCR Reaktionen häufig als "Schmier" auftretende Hintergrund deutlich reduziert.

### Beispiel 7

In diesem Beispiel wurde der Effekt von RNA als "Futter" im Sinne der Erfindung auf die High Fidelity PCR (PCR Amplifikation von β-Actin) untersucht. Es wurde kommerziell erhältliche Transfer-RNA (tRNA, R8508, Sigma GmbH, Taufkirchen bei München, Deutschland) sowie ribosomale RNA (rRNA, R6750, Sigma) als "Futter" der High-Fidelity PCR zugesetzt.

Der Ansatz und die Durchführung der High Fidelity PCR waren wie folgt. Für die PCR-Reaktionen wurde ProofStart DNA-Polymerase verwendet (QIAGEN, Katalog Nummer 202205). Jeder Reaktionsansatz enthielt 1x ProofStart PCR-Buffer, 1 U ProofStart DNA-Polymerase, je 1 µM β-Actin Vorwärts- und -Rückwärts-Primer und je 0,3 mM dNTPs (dATP, dCTP, dGTP, dTTP) in einem Reaktionsvolumen von 20 µl. Den Dreifachansätzen wurden jeweils 100 ng, 10 ng, 1 ng, 0,1 ng bzw. 0,01 ng K562 cDNA (aus K562 Zellen, siehe allgemeine Methoden) und in einer Einfachbestimmung eine vergleichbare Menge Wasser als Negativkontrolle (NTC: "No-Template-Control") zugesetzt. Zusätzlich wurden als "Futter" im Sinne der Erfindung jeweils t-RNA in einer Konzentration von 4 ng, 40 ng, 400 ng bzw. 4000 ng pro 20 µl Reaktionsansatz bzw. r-RNA in einer Konzentration von 4 ng, 40 ng bzw. 400 ng pro 20 µl Reaktionsansatz zugesetzt. Als Kontrolle wurden jeweils entsprechende Reaktionen ohne "Futter"-RNA mitgeführt (Beschriftung in Fig. 7A: "Kontrolle"). Während der Vorbereitung der PCR Reaktionen wurden die Proben gekühlt.

Das PCR Protokoll bestand aus einer initialen Reaktivierung der ProofStart DNA-Polymerase ("Hot-Start") für 5 min bei 95°C, gefolgt von 35 Zyklen mit 30 s bei 94°C, 60 s bei 61°C und 1 min 30 s bei 72°C sowie einem abschließenden Schritt für 10 min bei 72°C ("final extension"). Die nachfolgenden Sequenzen von Primern sind in 5'-3' Richtung angegeben.

### PCR Primer-Sequenzen:

β-Actin Vorwärts: TCA CCC ACA CTG TGC CCA TCT ACG A
β-Actin Rückwärts: CAG CGG AAC CGC TCA TTG CCA ATG G

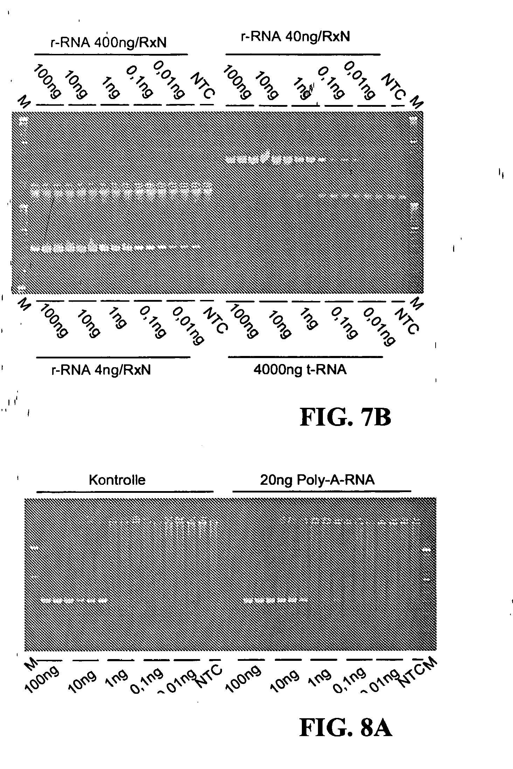

5 µl jeder PCR-Reaktion wurden auf einem mit Ethidiumbromid gefärbten Agarosegel (2 %) analysiert. Als Größenstandard diente eine 100 bp Leiter (Invitrogen, 15628-050). Die Fig. 7A und 7B zeigen Agarosegel-Analysen zur Untersuchung des Effekts von RNA als "Futter" im Sinne der Erfindung auf die PCR Amplifikation von β-Actin. M = Marker.

Überraschenderweise eignet sich RNA als "Futter" im Sinne der Erfindung zur Verbesserung von Ausbeute und Sensitivität der High Fidelity PCR. Bei Verwendung von t-RNA (Fig. 7A) konnte die Sensitivität bis zu 100-fach verbessert werden. Bei Verwendung von rRNA (Fig. 7B) konnte die Sensitivität sogar bis zu 1.000-fach verbessert werden. Gleichzeitig wurde bei Zusatz von RNA (Fig. 7A und B) der bei High-Fidelity PCR Reaktionen häufig als "Schmier" auftretende Hintergrund deutlich reduziert.

### Beispiel 8

In diesem Beispiel wurde der Effekt von RNA als "Futter" im Sinne der Erfindung auf die High Fidelity PCR (die PCR Amplifikation von β-Actin) untersucht. Es wurde kommerziell erhältliche Poly-A-RNA (QIAGEN, 1010373) als "Futter" der High-Fidelity PCR zugesetzt. Es handelt sich dabei um ein Homo-A-Polymer, welcher ein heterogenes Gemisch aus unterschiedlich langen poly(A)-Molekülen enthält (Hauptanteil zwischen 0.2 kb und 5.0 kb).

Der Ansatz und die Durchführung der High Fidelity PCR waren wie folgt. Für die PCR Reaktionen wurde ProofStart DNA-Polymerase verwendet (QIAGEN, Katalog Nummer 202205). Jeder Reaktionsansatz enthielt 1xProofStart PCR-Buffer, 2,5 U ProofStart DNA-Polymerase, je 1 µM β-Actin-Vorwärts- und Rückwärts-Primer und je 0,3 mM dNTPs (dATP, dCTP, dGTP, dTTP) in einem Reaktionsvolumen von 50 µl. Den Dreifachansätzen wurden jeweils 100 ng, 10 ng, 1 ng, 0,1 ng bzw. 0,01 ng pro Reaktion cDNA (aus K562 Zellen, siehe allgemeine Methoden im Anschluß an die Beispiele) und in Einzelbestimmung eine vergleichbare Menge Wasser als Negativkontrolle (NTC: "No-Template-Control") zugesetzt. Zusätzlich wurden als "Futter" Poly-A-RNA im Sinne der Erfindung jeweils in einer Konzentration von 20 ng, 200 ng, 2000 ng, oder 20.000 ng pro 50 µl Reaktionsansatz zugesetzt. Als Kontrolle wurden jeweils entsprechende Reaktionen ohne "Futter"-RNA mitgeführt (Beschriftung in Fig. 8A: "Kontrolle"). Während der Vorbereitung der PCR Reaktionen wurden die Proben gekühlt.

Das PCR Protokoll bestand aus einer initialen Reaktivierung der ProofStart DNA-Polymerase ("Hot-Start") für 5 min bei 95°C, gefolgt von 40 Zyklen mit 30 s bei 94°C, 60 s bei 61°C und 1 min 30 s bei 72°C sowie einem abschließenden Schritt für 10 min bei 72°C ("final extension"). Die nachfolgenden Sequenzen von Primern sind in 5'-3' Richtung angegeben.

### PCR Primer-Sequenzen:

β-Actin Vorwärts: TCA CCC ACA CTG TGC CCA TCT ACG A
β-Actin Rückwärts: CAG CGG AAC CGC TCA TTG CCA ATG G

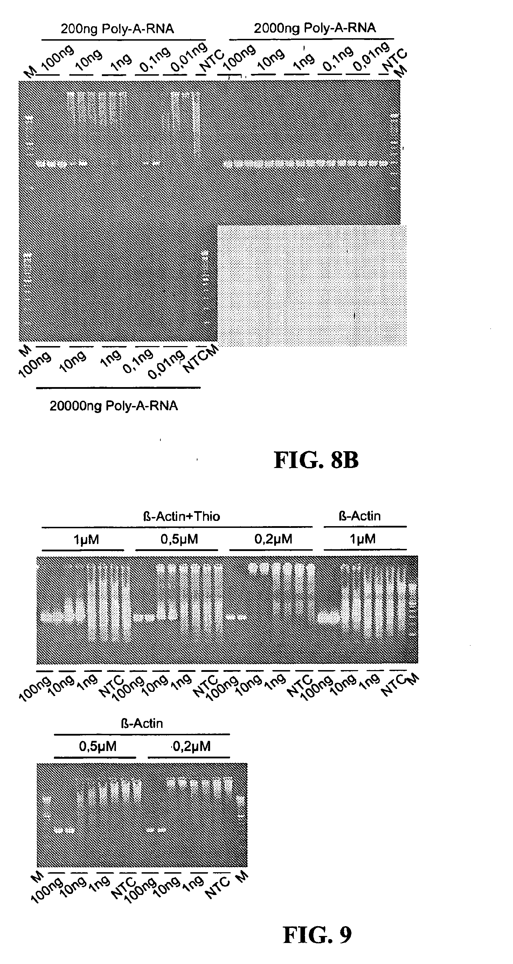

10 µl jeder PCR Reaktion wurden auf einem mit Ethidiumbromid gefärbten Agarosegel (1 %) analysiert. Als Größenstandard diente eine 100 bp Leiter (Invitrogen, 15628-050). Die Fig. 8A und 8B zeigen Agarosegel-Analysen zur Untersuchung des Effekts des Zusatzes von Poly-A-RNA auf die PCR-Amplifikation von β-Actin.

Überraschenderweise eignet sich auch Poly-A-RNA als "Futter" im Sinne der Erfindung zur Verbesserung von Ausbeute und Sensitivität der High Fidelity PCR. Bei Verwendung von Poly-A-RNA konnte die Sensitivität bis zu 10.000-fach verbessert werden. Gleichzeitig wurde bei Zusatz von RNA (vergl. Fig. 8A und B) der bei High-Fidelity PCR Reaktionen häufig als "Schmier" auftretende Hintergrund deutlich reduziert.

### Beispiel 9

In diesem Beispiel sollte der Effekt von PCR Primern, die zwischen der letzten und vorletzten 3'-Base ein Phosphothioat im Zucker-Phosphat Rückgrat der DNA tragen, auf die High Fidelity PCR (die Amplifikation von β-Actin) untersucht und mit Standard PCR Primern verglichen werden. In der Literatur (Skerra, A., PhosphoroPhosphothioat primers improve the amplification of DNA sequences by DNA polymerases with proofreading activity, Nucleic Acids Res, 1992, 20(14), 3551-3554) wurde eine positiver Effekt von PCR Primern, die zwischen der letzten und vorletzten 3'-Base ein Phosphothioat im Zucker-Phosphat Rückgrat tragen, beschrieben. In diesem Experiment sollte der Effekt solcher Primer im Vergleich zu Standard Primern auf die Amplifikation des in Beispiel 4 bis 8 verwendeten β-Actin PCR Systems untersucht werden. Der Effekt der Primer wurde in einem Konzentrationsbereich von 0,2 µM bis 1 µM getestet.

Der Ansatz und die Durchführung der High Fidelity PCR waren wie folgt. Für die PCR-Reaktionen wurde ProofStart DNA-Polymerase verwendet (QIAGEN, Katalog Nummer 202205). Jeder Reaktionsansatz enthielt 1x ProofStart PCR Buffer, 2,5 U ProofStart DNA Polymerase, je 0,2 µM, 0,5 µM oder 1 µM β-Actin Vorwärts-Thio und Rückwärts-Thio-Primer und je 0,3 mM dNTPs (dATP, dCTP, dGTP, dTTP) in einem Reaktionsvolumen von 50 µl. Den Zweifachansätzen wurde jeweils 100 ng, 10 ng, 1 ng K562 cDNA und ebenfalls in Zweifachansätzen eine vergleichbare Menge Wasser als Negativkontrolle (NTC: "No-Template-Control") zugegeben. Als Kontrolle wurden jeweils entsprechende PCR-Reaktionen mit den beiden Standard Primern (β-Actin-Vorwärts bzw. -Rückwärts) je 0,2 µM, 0,5 µM oder 1 µM mitgeführt (Beschriftung: "Standard Primer"). Während der Vorbereitung der PCR Reaktionen wurde die Proben gekühlt.

Das PCR Protokoll bestand aus einer initialen Reaktivierung der ProofStart DNA-Polymerase ("Hot-Start") für 5 min bei 95°C, gefolgt von 35 Zyklen mit 30 s bei 94°C, 60 s bei 61°C und 1min 30 s bei 72°C sowie einem abschließenden Schritt für 10 min bei 72°C ("final extension"). Die nachfolgenden Sequenzen von Primern sind in 5'-3' Richtung angegeben.

### PCR Primer-Sequenzen:

β-Actin Vorwärts: TCA CCC ACA CTG TGC CCA TCT ACG A
β-Actin Rückwärts: CAG CGG AAC CGC TCA TTG CCA ATG G
β-Actin Vorwärts-Thio: TCA CCC ACA CTG TGC CCA TCT ACG *A
β-Actin Rückwärts-Thio: CAG CGG AAC CGC TCA TTG CCA ATG *G (*: Phosphothioat im Rückgrat)

10 µl jeder PCR Reaktion wurden auf einem mit Ethidiumbromid gefärbten Agarosegel (2 %) analysiert. Als Größenstandard diente eine 100 bp Leiter (Invitrogen, 15628-050). Fig. 9 zeigt eine Agarosegel-Analyse zur Untersuchung des Effekts von PCR-Primern, die zwischen der letzten und vorletzten 3'-Base ein Phosphothioat im Zucker-Phosphat-Rückgrat der DNA tragen, auf die Amplifikation von β-Actin im Vergleich zu Standard PCR Primern. M = Marker.

Durch Verwendung von PCR-Primern, die zwischen der letzten und vorletzten 3'-Base ein Phosphothioat im Zucker-Phosphat Rückgrat tragen, konnte eine leichte Verbesserung der Ausbeute und Sensitivität im Vergleich zu Standard Primern erzielt werden. die Erhöhung der Sensitivität lag bei maximal 10-fach. Im Gegensatz dazu konnte die Sensitivität bei dem gleichen PCR-System in den Beispielen 4 bis 8 um bis zu 10.000-fach gesteigert werden.

Gleichzeitig wurde bei Verwendung der PCR-Primer, die zwischen der letzten und vorletzten 3'-Base ein Phosphothioat im Zucker-Phosphat Rückgrat der DNA tragen, keine Reduktion des bei High-Fidelity PCR Reaktionen häufig als "Schmier" auftretende Hintergrunds beobachtet. Im Vergleich zum Stand der Technik stellt die Verwendung von "Futter" im Sinne der Erfindung eine signifikante Verbesserung der High-Fidelity PCR dar.

### Beispiel 10

Dieses Beispiel betrifft den Effekt von Haarnadel "Futter" Oligonukleotiden auf die PCR Amplifikation eines 2 kb Fragments aus dem murinen PKC Genlokus unter Verwendung des Enzyms VentR. Speziell wurde in diesem Beispiel der Effekt des Haarnadel "Futter" DNA-Oligonukleotids GAP P78 3'p-Thio auf die High Fidelity PCR unter Verwendung einer High Fidelity DNA-Polymerase aus verschiedenen Archaebakterien-Gattungen untersucht. Das Enzym VentR (New England Biolabs, M0254S) stammt ursprünglich aus *Thermococcus spec.* während die in den vorhergehenden Beispielen verwendete ProofStart Polymerase ursprünglich aus *Pyrococcus spec.* isoliert wurde. Im Vergleich zur Proof-Start Polymerase fehlt der VentR Polymerase zusätzlich auch ein PCR Hot-Start. Das Oligonukleotid GAP P78 3'p-Thio wurde bereits in Beispiel 2 näher beschrieben.

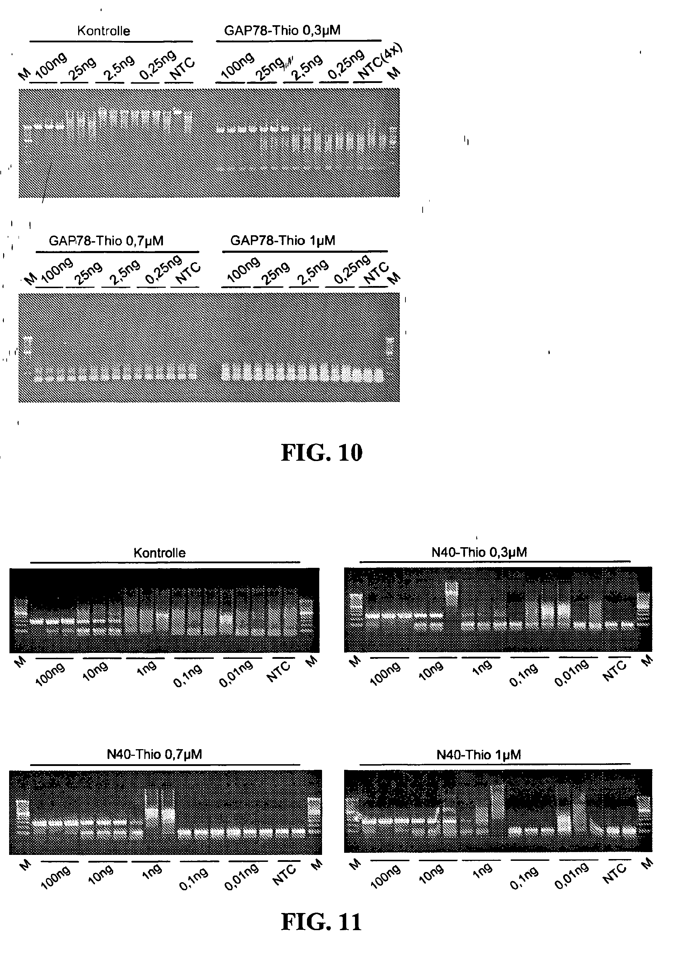

Der Ansatz und die Durchführung der High Fidelity PCR waren wie folgt. Für die PCR-Reaktionen wurde VentR-Polymerase verwendet (New England Biolabs, Katalog Nummer M0254S). Jeder Reaktionsansatz enthielt 1x ThermoPol Reaction PCR-Buffer, 2,5 U VentR DNA-Polymerase, je 0,4 µM MPUC-Vorwärts- und -Rückwärts-Primer und je 0,4 mM dNTPs (dATP, dCTP, dGTP, dTTP) in einem Reaktionsvolumen von 50 µl. Den Dreifachansätzen wurden jeweils 100 ng, 25 ng, 2,5 ng bzw. 0,25 ng 3T3 DNA sowie eine vergleichbare Menge Wasser als Negativkontrolle (NTC: "No-Template-Control") zugesetzt. Zusätzlich wurde als "Futter"-Oligonukleotid im Sinne der Erfindung GAP P78 3'p-Thio in einer Konzentration von 0,3 µM, 0,7 µM oder 1 µM zugesetzt. Als Kontrolle wurden jeweils entsprechende Reaktionen ohne "Futter"-Oligonukleotid mitgeführt (Beschriftung in Fig. 10: "Kontrolle). Während der Vorbereitung der PCR Reaktionen wurden die Proben gekühlt.

Das PCR Protokoll bestand aus 40 Zyklen mit 30 s bei 94°C, 60 s bei 61°C und 1 min 30 s bei 72°C sowie einem abschließenden Schritt für 10 min bei 72°C ("final extension"). Die nachfolgenden Sequenzen von Oligonukleotiden und Primern sind in 5'-3' Richtung angegeben.

### PCR Primer-Sequenzen:

MPUC 3: GCT GCT TGA AGA AAC GAG CGG TG
MPUC5+258: CTG CAC CTT CTG GAA TTC CGA CTC

### Sequenzen der "Futter" Oligonukleotide:

GAP P78 3'p-Thio: GCG TCA AAG GTG GAG GAG TGG GTG TCG CTG TTG AAG TCA TGA CTT CAA CAG CGA CAC CCA CTC CTC CAC CTT TGA CG*C [Phosp-Q] ([Phosp-Q]: 3'-Phosphat; *: Phosphothioat im Rückgrat)

10 µl jeder PCR Reaktion wurden auf einem mit Ethidiumbromid gefärbten 2 % Agarosegel analysiert. Als Größenstandard diente eine 100 bp Leiter (Invitrogen, 15628-050). Fig. 10 zeigt eine Agarosegel Analyse zur Untersuchung des Effekts von Haarnadel- "Futter"-Oligonukleotiden auf die PCR-Amplifikation eines 2 kb Fragments aus dem murinen PKC Genlokus unter Verwendung des Enzyms VentR. M= Marker.

Die Verwendung des "Futters" im Sinne der Erfindung ist auch mit einer High-Fidelity DNA Polymerase aus einer anderen Archaen-Gattung kompatibel und erlaubt eine Verbesserung der Ausbeute und Sensitivität der High-Fidelity PCR. Der Zusatz des Haarnadel-"Futter"-Oligonukleotids GAP P78 3'p-Thio führt hier zu einer bis Verbesserung der Sensitivität um das bis zu 40-fache für ein 2 kb PCR Produkt.

### Beispiel 11

In diesem Beispiel wurde der Effekt des "Futter"-DNA-Oligonukleotids N40-Thio auf die High Fidelity PCR (PCR Amplifikation von β-Actin) unter Verwendung einer High Fidelity DNA-Polymerase aus verschiedenen Archaebakterien-Gattungen untersucht. Die Polymerase VentR (New England Biolabs, M0254S) stammte ursprünglich aus *Thermococcus spec.* während die ProofStart Polymerase ursprünglich aus *Pyrococcus spec.* isoliert wurde. Im Vergleich zur Proof-Start Polymerase fehlt der VentR Polymerase zusäzlich auch PCR Hot-Start. Bei dem Futter-Oligonukleotid handelt sich um ein einzelsträngiges Oligonukleotid mit zufälliger Sequenz aus A, C, G und T (N), welches anstatt der 3'-OH-Gruppe ein 3'-Phosphat trägt und zusätzlich zwischen der letzten und vorletzten 3'-Base ein Phosphothioat im Zucker-Phosphat Rückgrat der DNA enthält.

Der Ansatz und die Durchführung der High Fidelity PCR waren wie folgt. Für die PCR-Reaktionen wurde VentR Polymerase verwendet (New England Biolabs, Katalog Nummer M0254S). Jeder Reaktionsansatz enthielt 1x ThermoPol Reaction PCR-Buffer, 1,25 U Vent R DNA-Polymerase, je 0,4 µM β-Actin-Vorwärts- und -Rückwärts-Primer und je 0,4 mM dNTPs (dATP, dCTP, dGTP, dTTP) in einem Reaktionsvolumen von 25 µl. Den Dreifachansätzen wurden jeweils 100, 10, 1, 0,1 bzw. 0,01 ng K562 cDNA sowie eine vergleichbare Menge Wasser als Negativkontrolle (NTC: "No-Template-Control") zugesetzt. Zusätzlich wurden als "Futter"-Oligonukleotid N40-Thio im Sinne der Erfindung jeweils in einer Konzentration von 0,3 µM, 0,7 µM oder 1 µM zugesetzt. Als Kontrolle wurden jeweils entsprechende Reaktionen ohne "Futter"-Oligonukleotid mitgeführt (Beschriftung in Fig. 11: "Kontrolle"). Während der Vorbereitung der PCR Reaktionen wurden die Proben gekühlt.

Das PCR Protokoll bestand aus 40 Zyklen mit 30 s bei 94°C, 60 s bei 61°C und 1 min 30 s bei 72°C sowie einem abschließenden Schritt für 10 min bei 72°C ("final extension"). Die nachfolgenden Sequenzen von Oligonukleotiden und Primern sind in 5'-3' Richtung angegeben.

### PCR Primer-Sequenzen:

β-Actin Vorwärts: TCA CCC ACA CTG TGC CCA TCT ACG A
β-Actin Rückwärts: CAG CGG AAC CGC TCA TTG CCA ATG G

### Sequenzen der "Futter" Oligonukleotide:

N40-Thio: NNN NNN NNN NNN NNN NNN NNN NNN NNN NNN NNN NNN NN*N [Phosp-Q] (N: A, C, G, T; [Phosp-Q]: 3'-Phosphate; *: Phosphothioat im Rückgrat)

10 µl jeder PCR-Reaktion wurden auf einem mit Ethidiumbromid gefärbten Agarosegel (1 %) analysiert. Als Größenstandard diente eine 100 bp Leiter (Invitrogen, 15628-050). Fig. 11 zeigt eine Agarosegel-Analyse zur Untersuchung des Effekst von "Futter" Oligonukleotiden auf die PCR-Amplifikation von β-Actin unter Verwendung des Enzyms VentR. M = Marker.

Der Zusatz des "Futter" Oligonukleotids N40-Thio führt hier zu einer Verbesserung der Ausbeute sowie einer Verbesserung der Sensitivität um den Faktor 10. Es eignet sich demnach als "Futter"-Oligonukleotid im Sinne dieser Erfindung. ,

### Beispiel 12

In diesem Beispiel wurde der Effekt des "Futter"-DNA-Oligonukleotids GAP45-Thio auf die High Fidelity PCR (PCR Amplifikation eines 2 kb Fragments aus dem murinen PKC Genlokus) unter Verwendung einer High Fidelity DNA-Polymerase aus verschiedenen Gattungen von Archaebakterien untersucht. Das verwendete Enzym VentR (New England Biolabs, M0254S) stammte ursprünglich aus *Thermococcus spec.,* während die ProofStart Polymerase ursprünglich aus *Pyrococcus spec.* isoliert wurde. Das Oligonukleotid GAP45-Thio wurde bereits in Beispiel 5 näher beschrieben.

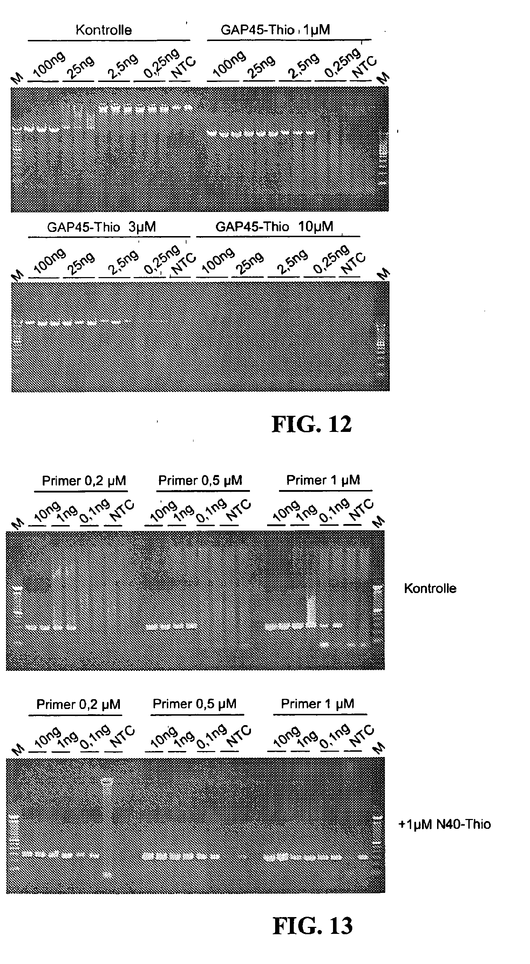

Der Ansatz und die Durchführung der High Fidelity PCR waren wie folgt. Für die PCR-Reaktionen wurde die VentR Polymerase verwendet (New England Biolabs, Katalog Nummer M0254S). Jeder Reaktionsansatz enthielt 1x ThermoPol Reaction PCR-Buffer, 1,25 U VentR DNA-Polymerase, je 0,4 µM MPUC Vorwärts- und -Rückwärts-Primer und je 0,4 mM dNTPs (dATP, dCTP, dGTP, dTTP) in einem Reaktionsvolumen von 25 µl. Den Dreifachansätzen wurden jeweils 100, 25, 2,5, bzw. 0,25 ng/RxN und in Zweifachansätzen eine vergleichbare Menge Wasser als Negativkontrolle (NTC: "No-Template-Control") hinzugefügt. Zusätzlich wurden als "Futter"-Oligonukleotid im Sinne der Erfindung GAP45-Thio in einer Konzentration von 0,3 µM, 0,7 µM oder 1 µM zugesetzt. Als Kontrolle wurden jeweils entsprechende Reaktionen ohne "Futter"-Oligonukleotid mitgeführt (Beschriftung in Fig. 12: "Kontrolle"). Während der Vorbereitung der PCR-Reaktionen wurden die Proben gekühlt.

Das PCR Protokoll bestand aus 40 Zyklen mit 30 s bei 94°C, 60 s bei 61°C und 1 min 30 s bei 72°C sowie einem abschließenden Schritt für 10 min bei 72°C ("final extension"). Die nachfolgenden Sequenzen von Oligonukleotiden und Primern sind in 5'-3' Richtung angegeben.

### PCR Primer-Sequenzen:

MPUC 3: GCT GCT TGA AGA AAC GAG CGG TG
MPUC5+258: CTG CAC CTT CTG GAA TTC CGA CTC

### Sequenzen der "Futter" Oligonukleotide:

GAP45-Thio: GCG TCA AAG GTG GAG GAG TGG GTG TCG CTG TTG AAG TCA GAG GA*G [Phosp-Q] ([Phosp-Q]: 3'-Phosphat; *: Phosphothioat im Rückgrat)

10 µl jeder PCR Reaktion wurden auf einem mit Ethidiumbromid gefärbten Agarosegel (2 %) analysiert. Als Größenstandard diente eine 100 bp Leiter (Invitrogen, 15628-050). Fig. 12 zeigt den Effekt von "'Futter"-Oligonukleotiden auf die PCR-Amplifikation eines 2 kb Fragments aus dem murinen PKC Genlokus unter Verwendung des Enzyms VentR. M=Marker.

Bei Verwendung des "Futter" Oligonukleotids GAP45-Thio konnte die Sensitivität bis zu 400-fach verbessert werden. Gleichzeitig wurde der bei High-Fidelity PCR Reaktionen häufig als "Schmier" auftretende Hintergrund deutlich reduziert.

### Beispiel 13

In diesem Beispiel wurde der Effekt von des "Futter"-Oligonukleotids N40-Thio in Verbindung mit variablen Konzentrationen der PCR-Primern auf die Amplifikation von β-Actin analysiert. Speziell wurde in diesem Beispiel der Effekt des "Futter"-DNA-Oligonukleotids N40-Thio auf die High Fidelity PCR in Gegenwart variabler Konzentrationen der PCR-Primer untersucht und mit Reaktionen ohne "Futter"-Oligonukleotid verglichen. Das verwendete "Futter"-Oligonukleotid N40-Thio wurde bereits in Beispiel 11 näher beschrieben. Der Effekt der Primer-Konzentration wurde in einem Konzentrationsbereich von 0,2 µM bis 1 µM getestet.

Der Ansatz und die Durchführung der High Fidelity PCR waren wie folgt. Für die PCR-Reaktionen wurde ProofStart DNA-Polymerase verwendet (QIAGEN, Katalog Nummer 202205). Jeder Reaktionsansatz enthielt 1x ProofStart PCR Buffer, 1,25 U ProofStart DNA-Polymerase, je 0,2, 0,5 bzw. 1 µM β-Actin Vorwärts- und -Rückwärts-Primer und je 0,3 mM dNTPs (dATP, dCTP, dGTP, dTTP) in einem Reaktionsvolumen von 25 µl. Den Zweifachansätzen wurden jeweils 1 ng, 0,1 ng bzw. 0,01 ng pro Reaktion K562 cDNA (aus K562 Zellen, siehe allgemeine Methoden am Ende der Beispiele) und in Einzelbestimmung eine vergleichbare Menge Wasser als Negativkontrolle (NTC: "No-Template-Control") zugesetzt. Zusätzlich wurde als "Futter"-Oligonukleotid im Sinne der Erfindung N40-Thio in einer Konzentration von 1 µM zugesetzt. Als Kontrolle wurden jeweils entsprechende Reaktionen ohne "Futter"-Oligonukleotid mitgeführt (Beschriftung in Fig. 13: "Kontrolle"). Während der Vorbereitung der PCR Reaktionen wurden die Proben gekühlt

Das PCR Protokoll bestand aus einer initialen Reaktivierung der ProofStart DNA-Polymerase ("Hot-Start") für 5 min bei 95°C, gefolgt von 40 Zyklen mit 30 s bei 94°C, 60 s bei 61°C und 1 min 30 s bei 72°C sowie einem abschließenden Schritt für 10 min bei 72°C ("final extension"). Die nachfolgenden Sequenzen von Oligonukleotiden und Primern sind in 5'-3'-Richtung angegeben.

### PCR Primer-Sequenzen:

β-Actin Vorwärts: TCA CCC ACA CTG TGC CCA TCT ACG A
β-Actin Rückwärts: CAG CGG AAC CGC TCA TTG CCA ATG G

### Sequenzen der "Futter" Oligonukleotide:

N40-Thio: NNN NNN NNN NNN NNN NNN NNN NNN NNN NNN NNN NNN NN*N [Phosp-Q] (N: A, C, G, T; [Phosp-Q]: 3'-Phosphat; *: Phosphothioat im Rückgrat)

10 µl jeder PCR Reaktion wurden auf einem mit Ethidiumbromid gefärbten Agarosegel (2 %) analysiert. Als Größenstandard diente eine 100 bp Leiter (Invitrogen, 15628-050). Fig. 13 zeigt den Effekt von "Futter"-Oligonukleotid N40-Thio in Verbindung mit blockierten PCR-Primem auf die Amplifikation von β-Actin. M=Marker.

Auch bei variablen Primer-Konzentrationen wurde eine Verbesserung der Ausbeute und Sensitivität in Gegenwart eines "'Futter"-Oligonukleotids erzielt. Gleichzeitig wurde der bei High-Fidelity PCR Reaktionen häufig als "Schmier" auftretende Hintergrund deutlich reduziert.

### Allgemeine Methoden

**I. Template Nukleinsäure:**
   **1. Genomische DNA:** Die verwendete humane genomische DNA (HuDNA bzw. gDNA) wurde mit einem QIAamp DNA Blood Maxi Kit (QIAGEN, Katalog Nummer 51192) oder einem FlexiGene DNA Kit (Qiagen, Katalog Nummer 51204) aus Blut isoliert. Die murine genomische DNA wurde aus NIH-3T3 Zellen mit einem DNeasy Tissue Kit (Qiagen, Katalog Nummer 69504) gewonnen. Die Konzentration der isolierten DNA wurde anschließend photometrisch (OD260) bestimmt.
   **2. cDNA:** Steht für "complementary DNA" und ist eine Bezeichnung für die einzel- oder doppelsträngige DNA-Kopie eines RNA-Moleküls. I
**II. Herstellung mittels reverser Transkription**
   **1. RNA:** Gesamt-RNA aus K562 Zellen wurde mit einem RNeasy Midi Kit (QIAGEN, Katalog Nummer 75142) isoliert.
   **2. Reverse Transkription:** Zur Herstellung von cDNA wurden 1µg Gesamt-RNA mit einem Omniscript RT Kit (QIAGEN, Katalog Nummer 205110) unter Verwendung einer Mischung aus Random und Oligo-dT(15) Primern (Random Octamer 10 µM, Oligonukleotid dT(15) 1 µM) entsprechend den Angaben im mitgelieferten Handbuch revers transkribiert.

## Patentansprüche

1. Polymerase-Kettenreaktions-Verfahren, bei dem eine Template-Nukleinsäure, mindestens ein Primer, Deoxyribonukleosidtriphosphate sowie eine Polymerase mit Proofreading-Aktivität eingesetzt werden, **dadurch gekennzeichnet, dass** bei der Polymerase-Kettenreaktion mindestens ein Zielsubstrat zugesetzt wird, wobei das Zielsubstrat eine Nukleinsäure ist, wobei weiterhin das Zielsubstrat unter Polymerase-Kettenreaktions-Bedingungen doppelsträngige Strukturen ausbildet, und wobei das Zielsubstrat nicht amplifiziert wird, und wobei ferner durch das Zielsubstrat der Abbau der Primer und/oder der Template-DNA durch die Proofreading-Aktivität der Polymerase reduziert wird.

2. Polymerase-Kettenreaktions-Verfahren nach Anspruch 1, **dadurch gekennzeichnet, dass** das mindestens eine Zielsubstrat die 3'-5'-Exonukleaseaktivität der Polymerase mit Proofreading-Aktivität partiell oder vollständig inhibiert.

3. Polymerase-Kettenreaktions-Verfahren nach Anspruch 1 oder 2, **dadurch gekennzeichnet, dass** das Zielsubstrat ein einzelsträngiges Oligonukleotid ist.

4. Polymerase-Kettenreaktions-Verfahren nach Anspruch 3, **dadurch gekennzeichnet, dass** die Nichtverlängerbarkeit des einzelsträngigen Oligonukleotids an seinem 3'-Ende durch eine Modifikation bewirkt wird.

5. Polymerase-Kettenreaktions-Verfahren nach Anspruch 4, **dadurch gekennzeichnet, dass** als Modifikation am 3'-Ende des einzelsträngigen Oligonukleotids die OH-Gruppe durch eine Phosphatgruppe oder einen anderen Rest ersetzt ist oder die Nichtverlängerbarkeit durch ein Didesoxy-Nukleotid, eine oder mehrere inverse Base(n), RNA, abasische Sites, Spacer, Farbstoffe, Quencher-Reste, wie z.B. Black Hole Quencher, Dabcyl, Minor-Groove-Binder, modifizierte Basen, wie z.B. Super-Basen oder halogenierte Basen, oder geeignete Basen-Analoga erreicht wird.

6. Polymerase-Kettenreaktions-Verfahren nach einem der Ansprüche 3 bis 5, **dadurch gekennzeichnet, dass** das Rückgrat des Oligonukleotids modifiziert ist.

7. Polymerase-Kettenreaktions-Verfahren nach Anspruch 6, **dadurch gekennzeichnet, dass** das Rückgrat des Oligonukleotids zumindest am 3'-Ende zwischen der letzten und der vorletzten Base modifiziert ist.

8. Polymerase-Kettenreaktions-Verfahren nach einem der Ansprüche 6 oder 7, **dadurch gekennzeichnet, dass** das Phosphat im Rückgrat des Oligonukleotids durch Phosphothioat ersetzt ist.

9. Polymerase-Kettenreaktions-Verfahren nach einem der Ansprüche 3 bis 8, **dadurch gekennzeichnet, dass** das Oligonukleotid zu der Template-Nukleinsäure zumindest partiell komplementär ist, so dass sich ein DNA-Doppelstrang ausbilden kann.

10. Polymerase-Kettenreaktions-Verfahren nach einem der Ansprüche 3 bis 8, **dadurch gekennzeichnet, dass** das Oligonukleotid eine Zufallssequenz aufweist.

11. Polymerase-Kettenreaktions-Verfahren nach einem der Ansprüche 3 bis 10, **dadurch gekennzeichnet, dass** das Oligonukleotid Basen, ausgewählt aus der Gruppe Adenin (A), Cytosin (C), Guanin (G) und Thymin (T), universelle Basen (wie z.B. Inosin, 3-Nitropyrrol oder 5-Nitro-indol), ein oder mehrere Uracil, Methyl-Cytosin, Basen-Analoga oder modifizierte Basen enthält.

12. Polymerase-Kettenreaktions-Verfahren nach einem der Ansprüche 3 bis 11, **dadurch gekennzeichnet, dass** das Oligonukleotid eine oder mehrere PNA (Peptide Nucleic Acid) oder LNA (Locked Nucleic Acid) enthält.

13. Polymerase-Kettenreaktions-Verfahren nach einem der Ansprüche 3 bis 8 oder 10 bis 12, **dadurch gekennzeichnet, dass** das Oligonukleotid ein Homo-Oligonukleotid ist.

14. Polymerase-Kettenreaktions-Verfahren nach einem der Ansprüche 3 bis 13, **dadurch gekennzeichnet, dass** das Oligonukleotid eine Länge von 10 bis 100 Basen, vorzugsweise 12 bis 80, insbesondere 20 bis 45 Basen, aufweist.

15. Polymerase-Kettenreaktions-Verfahren nach einem der Ansprüche 3 bis 14, **dadurch gekennzeichnet, dass** das Oligonukleotid in der Polymerase-Kettenreaktion in einer Konzentration von 0,1 bis 20 M, vorzugsweise 0,2 bis 2,5 M, insbesondere 0,5 bis 1,5 M, vorliegt.

16. Polymerase-Kettenreaktions-Verfahren nach einem der Ansprüche 3 bis 15, **dadurch gekennzeichnet, dass** das Molekül-Verhältnis der Polymerase mit Proofreading-Aktivität zu dem Zielsubstrat zwischen 1:1 und 1:1000, vorzugsweise zwischen 1:1 und 1:500, und insbesondere zwischen 1:1 und 1:200, liegt.

17. Polymerase-Kettenreaktions-Verfahren nach Anspruch 1 oder 2, **dadurch gekennzeichnet, dass** mindestens eine Zielsubstrat ein doppelsträngiges Oligonukleotid ist.

18. Polymerase-Kettenreaktions-Verfahren nach Anspruch 17 **dadurch gekennzeichnet, dass** das Rückgrat des Oligonukleotids modifiziert ist.

19. Polymerase-Kettenreaktions-Verfahren nach Anspruch 18, **dadurch gekennzeichnet, dass** das Rückgrat des Oligonukleotids zumindest am 3'-Ende zwischen der letzten und der vorletzten Base modifiziert ist.

20. Polymerase-Kettenreaktions-Verfähren nach einem der Ansprüche 18 oder 19, **dadurch gekennzeichnet, dass** das Phosphat im Rückgrat des Oligonukleotids durch Phosphothioat ersetzt ist.

21. Polymerase-Kettenreaktions-Verfahren nach einem der Ansprüche 17 bis 20, **dadurch gekennzeichnet, dass** das Zielsubstrat eine Zielsequenz aufweist, die eine mit sich selbst komplementäre Haarnadelstruktur ausbilden kann.

22. Polymerase-Kettenreaktions-Verfahren nach Anspruch 21, **dadurch gekennzeichnet, dass** das Zielsubstrat mindestens eine Modifikation in der Basensequenz und/oder im Rückgrat der DNA aufweist.

23. Polymerase-Kettenreaktions-Verfahren nach einem der Ansprüche 17 bis 22,
**dadurch gekennzeichnet, dass** das Zielsubstrat zumindest während des Annealing-Schritts der Polymerase-Kettenreaktion einen DNA-Doppelstrang ausbildet.

24. Polymerase-Kettenreaktions-Verfahren nach Anspruch 23, **dadurch gekennzeichnet, dass** das Annealing bei einer Temperatur zwischen 40 und 70°C durchgeführt wird.

25. Polymerase-Kettenreaktions-Verfahren nach einem der Ansprüche 17 bis 24, **dadurch gekennzeichnet, dass** im Falle einer Haarnadelstruktur des Zielsubstrats die Länge des selbstkomplementären Bereichs zwischen 10 und 100 Basen, vorzugsweise 12 bis 80 Basen, insbesondere 20 bis 45 Basen, liegt.

26. Polymerase-Kettenreaktions-Verfahren nach einem der Ansprüche 17 bis 24, **dadurch gekennzeichnet, dass** die Länge des Zielsubstrats zwischen 20 und 200 Basen, vorzugsweise zwischen 24 und 160 Basen, liegt.

27. Polymerase-Kettenreaktions-Verfahren nach einem der Ansprüche 17 bis 26, **dadurch gekennzeichnet, dass** das Oligonukleotid in der Polymerase-Kettenreaktion in einer Konzentration von 0,05 bis 20 M, vorzugsweise von 0,2 bis 10 M, und insbesondere von 0,2 bis 2,5 M, vorliegt.

28. Polymerase-Kettenreaktions-Verfahren nach einem der Ansprüche 17 bis 27, **dadurch gekennzeichnet, dass** das Molekül-Verhältnis der Polymerase mit Proofreading-Aktivität zu dem Zielsubstrat zwischen 1:0,5 und 1:1000, vorzugsweise zwischen 1:0,5 und 1:500, und insbesondere zwischen 1:1 und 1:200 beträgt.

29. Polymerase-Kettenreaktions-Verfahren nach einem der Ansprüche 17 bis 28, **dadurch gekennzeichnet, dass** das 5'-Ende des Oligonukleotids eine Modifikation trägt.

30. Polymerase-Kettenreaktions-Verfahren nach Anspruch 1 oder 2, **dadurch gekennzeichnet, dass** das mindestens eine Zielsubstrat ein doppelsträngiges DNA-Molekül ist, das keine Bindungsstelle für den bei der Polymerase-Kettenreaktion verwendeten Primer enthält.

31. Polymerase-Kettenreaktions-Verfahren nach Anspruch 30, **dadurch gekennzeichnet, dass** das doppelsträngige DNA-Molekül an seinem 3'-Ende eine Modifikation aufweist.

32. Polymerase-Kettenreaktions-Verfahren nach Anspruch 31, **dadurch gekennzeichnet, dass** als Modifikation am 3'-Ende des doppelsträngigen DNA-Moleküls die OH-Gruppe durch eine Phosphatgruppe ersetzt ist.

33. Polymerase-Kettenreaktions-Verfahren nach einem der Ansprüche 30 bis 32, **dadurch gekennzeichnet, dass** das Rückgrat des DNA-Moleküls modifiziert ist.

34. Polymerase-Kettenreaktions-Verfahren nach Anspruch 33, **dadurch gekennzeichnet, dass** das Rückgrat des DNA-Moleküls zumindest am 3'-Ende zwischen der letzten und der vorletzten Base modifiziert ist.

35. Polymerase-Kettenreaktions-Verfahren nach einem der Ansprüche 33 oder 34, **dadurch gekennzeichnet, dass** das Phosphat im Rückgrat des DNA-Moleküls durch Phosphothioat ersetzt ist.

36. Polymerase-Kettenreaktions-Verfahren nach einem der Ansprüche 30 bis 35, **dadurch gekennzeichnet, dass** das Oligonukleotid ein zirkuläres DNA-Molekül ist.

37. Polymerase-Kettenreaktions-Verfahren nach Anspruch 1 oder 2, **dadurch gekennzeichnet, dass** das mindestens eine Zielsubstrat ein einzelsträngiges DNA-Molekül ist, das keine Bindungsstelle für den bei der Polymerase-Kettenreaktion verwendeten Primer enthält.

38. Polymerase-Kettenreaktions-Verfahren nach Anspruch 37, **dadurch gekennzeichnet, dass** das einzelsträngige DNA-Molekül an seinem 3'-Ende eine Modifikation aufweist.

39. Polymerase-Kettenreaktions-Verfahren nach Anspruch 38, **dadurch gekennzeichnet, dass** als Modifikation am 3'-Ende des einzelsträngigen DNA-Moleküls die OH-Gruppe durch eine Phosphatgruppe ersetzt ist.

40. Polymerase-Kettenreaktions-Verfahren nach einem der Ansprüche 37 bis 39, **dadurch gekennzeichnet, dass** das Rückgrat des DNA-Moleküls modifiziert ist.

41. Polymerase-Kettenreaktions-Verfahren nach Anspruch 40, **dadurch gekennzeichnet, dass** das Rückgrat des DNA-Moleküls zumindest am 3'-Ende zwischen der letzten und der vorletzten Base modifiziert ist.

42. Polymerase-Kettenreaktions-Verfahren nach einem der Ansprüche 40 oder 41, **dadurch gekennzeichnet, dass** das Phosphat im Rückgrat des DNA-Moleküls durch Phosphothioat ersetzt ist.

43. Polymerase-Kettenreaktions-Verfahren nach einem der Ansprüche 37 bis 42, **dadurch gekennzeichnet, dass** das Oligonukleotid ein zirkuläres DNA-Molekül ist.

44. Polymerase-Kettenreaktions-Verfahren nach einem der Ansprüche 30 bis 43, **dadurch gekennzeichnet, dass** das Verhältnis Polymerase zu Zielsubstratbasen zwischen 1:20 und 1:1000 ist.

45. Polymerase-Kettenreaktions-Verfahren nach einem der Ansprüche 30 bis 44, **dadurch gekennzeichnet, dass** als Zielsubstrat eine Schar von DNA-Molekülen mit verschiedenen Sequenzmotiven eingesetzt wird.

46. Polymerase-Kettenreaktions-Verfahren nach einem der Ansprüche 30 bis 45, **dadurch gekennzeichnet, dass** das Zielsubstrat zumindest während des Annealing-Schritts der Polymerase-Kettenreaktion einen DNA-Doppelstrang ausbildet.

47. Polymerase-Kettenreaktions-Verfahren nach Anspruch 46, **dadurch gekennzeichnet, dass** das Annealing bei einer Temperatur zwischen 40 und 70°C durchgeführt wird.

48. Polymerase-Kettenreaktions-Verfahren nach einem der Ansprüche 30 bis 47, **dadurch gekennzeichnet, dass** die Länge des Zielsubstrats bis zu 10.000 Basen beträgt.

49. Polymerase-Kettenreaktions-Verfahren nach einem der Ansprüche 30 bis 36, **dadurch gekennzeichnet, dass** das 5'-Ende der doppelsträngigen DNA eine Modifikation trägt.

50. Polymerase-Kettenreaktions-Verfahren nach Anspruch 1 oder 2, **dadurch gekennzeichnet, dass** das Zielsubstrat eine RNA ist.

51. Polymerase-Kettenreaktions-Verfahren nach Anspruch 50, **dadurch gekennzeichnet, dass** die RNA Didesoxynukleotide enthält.

52. Polymerase-Kettenreaktions-Verfahren nach Anspruch 50 oder 51, **dadurch gekennzeichnet, dass** die RNA an ihrem 3'-Ende eine Modifikation aufweist.

53. Polymerase-Kettenreaktions-Verfahren nach Anspruch 52, **dadurch gekennzeichnet, dass** als Modifikation am 3'-Ende der RNA die OH-Gruppe durch eine Phosphatgruppe ersetzt ist.

54. Polymerase-Kettenreaktions-Verfahren nach einem der Ansprüche 50 bis 53, **dadurch gekennzeichnet, dass** das Rückgrat der RNA modifiziert ist.

55. Polymerase-Kettenreaktions-Verfahren nach Anspruch 54, **dadurch gekennzeichnet, dass** das Rückgrat des Oligonukleotids zumindest am 3'-Ende zwischen der letzten und der vorletzten Base modifiziert ist.

56. Polymerase-Kettenreaktions-Verfahren nach einem der Ansprüche 54 oder 55, **dadurch gekennzeichnet, dass** das Phosphat im Rückgrat des Oligonukleotids durch Phosphothioat ersetzt ist.

57. Polymerase-Kettenreaktions-Verfahren nach einem der Ansprüche 50 bis 56, **dadurch gekennzeichnet, dass** die RNA aus einer Schar von RNA-Molekülen mit verschiedenen Sequenzmotiven besteht.

58. Polymerase-Kettenreaktions-Verfahren nach einem der Ansprüche 50 bis 57, **dadurch gekennzeichnet, dass** das RNA-Zielmolekül ein Homopolymer ist.

59. Polymerase-Kettenreaktions-Verfahren nach einem der Ansprüche 50 bis 58, **dadurch gekennzeichnet, dass** das Zielsubstrat zumindest während des Annealing-Schritts der Polymerase-Kettenreaktion einen DNA/RNA-Doppelstrang ausbildet.

60. Polymerase-Kettenreaktions-Verfahren nach Anspruch 59, **dadurch gekennzeichnet, dass** das Annealing bei einer Temperatur zwischen 40 und 70°C durchgeführt wird.

61. Polymerase-Kettenreaktions-Verfahren nach einem der Ansprüche 50 bis 60, **dadurch gekennzeichnet, dass** die Länge des Zielsubstrats zwischen 10 und 10.000 Basen liegt

62. Polymerase-Kettenreaktions-Verfahren nach einem der Ansprüche 50 bis 61, **dadurch gekennzeichnet, dass** das 5'-Ende der RNA eine Modifikation trägt.

63. Kit zur Durchführung eines Polymerase-Kettenreaktions-Verfahrens gemäß einem der Ansprüche 1 bis 62, wobei der Kit mindestens ein Zielsubstrat und weiterhin mindestens einen Primer, Deoxyribonukleosidtriphosphate, einen PCR-Puffer, eine Lösung mit Mg²⁺-Ionen, PCR-Additive und/oder eine Polymerase mit Proofreading-Aktivität enthält, wobei das Zielsubstrat eine Nukleinsäure ist, wobei weiterhin das Zielsubstrat unter Polymerase-Kettenreaktions-Bedingungen doppelsträngige Strukturen ausbildet, und wobei das Zielsubstrat nicht amplifiziert wird, und wobei ferner durch das Zielsubstrat der Abbau der Primer und/oder der Template-DNA durch die Proofreading-Aktivität der Polymerase reduziert wird.

64. Kit nach Anspruch 63, **dadurch gekennzeichnet, dass** die PCR-Additive ausgewählt sind aus Betain, Polyethylenglykol (PEG), Dextran, Glyzerin, Dimethylsulfoxid (DMSO) Bovine Serum Albumin (BSA), einzelsträngiges, DNA-bindendes Protein (single-strand DNA-binding-protein) und/oder (nicht-ionische) Detergenzien.

## Claims

1. Polymerase chain reaction method using a template nucleic acid, at least one primer, deoxyribonucleoside triphosphates and a polymerase having proofreading activity, **characterized in that** at least one target substrate is added in the polymerase chain reaction, wherein the target substrate is a nucleic acid, wherein furthermore the target substrate forms double-stranded structures under polymerase chain reaction conditions, and wherein the target substrate is not amplified, and wherein further the target substrate reduces degradation of the primers and/or the template DNA due to the proofreading activity of the polymerase.

2. Polymerase chain reaction method according to Claim 1, **characterized in that** the at least one target substrate partially or completely inhibits the 3'-5' exonuclease activity of the polymerase having proofreading activity.

3. Polymerase chain reaction method according to Claim 1 or 2, **characterized in that** the target substrate is a single-stranded oligonucleotide.

4. Polymerase chain reaction method according to Claim 3, **characterized in that** a modification causes the 3' end of the single-stranded oligonucleotide to be non-extendable.

5. Polymerase chain reaction method according to Claim 4, **characterized in that** the 3' end of the single-stranded oligonucleotide is modified by way of the OH group being replaced with a phosphate group or another residue, or the non-extensibility is achieved by means of a dideoxy nucleotide, one or more inverse base(s), RNA, abasic sites, spacers, dyes, quencher radicals such as, for example, black hole quencher, dabcyl, minor-groove binders, modified bases such as, for example, super bases or halogenated bases, or suitable base analogues.

6. Polymerase chain reaction method according to any of Claims 3 to 5, **characterized in that** the backbone of the oligonucleotide is modified.

7. Polymerase chain reaction method according to Claim 6, **characterized in that** the backbone of the oligonucleotide is modified at least at the 3' end between the last and the second-last base.

8. Polymerase chain reaction method according to either of Claims 6 and 7, **characterized in that** the phosphate in the backbone of the oligonucleotide is replaced with phosphothioate.

9. Polymerase chain reaction method according to any of Claims 3 to 8, **characterized in that** the oligonucleotide is at least partially complementary to the template nucleic acid, thereby enabling a DNA double strand to form.

10. Polymerase chain reaction method according to any of Claims 3 to 8, **characterized in that** the oligonucleotide has a random sequence.

11. Polymerase chain reaction method according to any of Claims 3 to 10, **characterized in that** the oligonucleotide comprises bases selected from the group consisting of adenine (A), cytosine (C), guanine (G) and thymine (T), universal bases (such as, for example, inosine, 3-nitropyrrole or 5-nitroindole), one or more uracil, methyl-cytosine, base analogues or modified bases.

12. Polymerase chain reaction method according to any of Claims 3 to 11, **characterized in that** the oligonucleotide comprises one or more PNAs (peptide nucleic acids) or LNAs (locked nucleic acids).

13. Polymerase chain reaction method according to any of Claims 3 to 8 or 10 to 12, **characterized in that** the oligonucleotide is a homo-oligonucleotide.

14. Polymerase chain reaction method according to any of Claims 3 to 13, **characterized in that** the oligonucleotide is from 10 to 100 bases, preferably 12 to 80, in particular 20 to 45, bases in length.

15. Polymerase chain reaction method according to any of Claims 3 to 14, **characterized in that** the concentration of the oligonucleotide in the polymerase chain reaction is from 0.1 to 20 M, preferably 0.2 to 2.5 M, in particular 0.5 to 1.5 M.

16. Polymerase chain reaction method according to any of Claims 3 to 15, **characterized in that** the molecular ratio of the polymerase having proofreading activity to the target substrate is between 1:1 and 1:1000, preferably between 1:1 and 1:500, and in particular between 1:1 and 1:200.

17. Polymerase chain reaction method according to Claim 1 or 2, **characterized in that** the at least one target substrate is a double-stranded oligonucleotide.

18. Polymerase chain reaction method according to Claim 17, **characterized in that** the backbone of the oligonucleotide is modified.

19. Polymerase chain reaction method according to Claim 18, **characterized in that** the backbone of the oligonucleotide is modified at least at the 3' end between the last and the second-last base.

20. Polymerase chain reaction method according to either of Claims 18 and 19, **characterized in that** the phosphate in the backbone of the oligonucleotide is replaced with phosphothioate.

21. Polymerase chain reaction method according to any of Claims 17 to 20, **characterized in that** the target substrate has a target sequence which can form a self-complementary hairpin structure.

22. Polymerase chain reaction method according to Claim 21, **characterized in that** the target substrate has at least one modification in the base sequence and/or in the backbone of the DNA.

23. Polymerase chain reaction method according to any of Claims 17 to 22, **characterized in that** the target substrate forms a DNA double strand at least during the annealing step of the polymerase chain reaction.

24. Polymerase chain reaction method according to Claim 23, **characterized in that** the annealing is carried out at a temperature of between 40 and 70°C.

25. Polymerase chain reaction method according to any of Claims 17 to 24, **characterized in that**, in the case of a hairpin structure of the target substrate, the length of the self-complementary region is between 10 and 100 bases, preferably 12 to 80 bases, in particular 20 to 45 bases.

26. Polymerase chain reaction method according to any of Claims 17 to 24, **characterized in that** the target substrate is between 20 and 200 bases, preferably between 24 and 160 bases, in length.

27. Polymerase chain reaction method according to any of Claims 17 to 26, **characterized in that** the concentration of the oligonucleotide in the polymerase chain reaction is from 0.05 to 20 M, preferably from 0.2 to 10 M, and in particular from 0.2 to 2.5 M.

28. Polymerase chain reaction method according to any of Claims 17 to 27, **characterized in that** the molecular ratio of the polymerase having proofreading activity to the target substrate is between 1:0.5 and 1:1000, preferably between 1:0.5 and 1:500, and in particular between 1:1 and 1:200.

29. Polymerase chain reaction method according to any of Claims 17 to 28, **characterized in that** the 5' end of the oligonucleotide carries a modification.

30. Polymerase chain reaction method according to Claim 1 or 2, **characterized in that** the at least one target substrate is a double-stranded DNA molecule which does not contain a binding site for the primer used in the polymerase chain reaction.

31. Polymerase chain reaction method according to Claim 30, **characterized in that** the 3' end of the double-stranded DNA molecule has a modification.

32. Polymerase chain reaction method according to Claim 31, **characterized in that** the 3' end of the double-stranded DNA molecule is modified by way of the OH group being replaced with a phosphate group.

33. Polymerase chain reaction method according to any of Claims 30 to 32, **characterized in that** the backbone of the DNA molecule is modified.

34. Polymerase chain reaction method according to Claim 33, **characterized in that** the backbone of the DNA molecule is modified at least at the 3' end between the last and the second-last base.

35. Polymerase chain reaction method according to either of Claims 33 and 34, **characterized in that** the phosphate in the backbone of the DNA molecule is replaced with phosphothioate.

36. Polymerase chain reaction method according to any of Claims 30 to 35, **characterized in that** the oligonucleotide is a circular DNA molecule.

37. Polymerase chain reaction method according to Claim 1 or 2, **characterized in that** the at least one target substrate is a single-stranded DNA molecule which does not contain a binding site for the primer used in the polymerase chain reaction.

38. Polymerase chain reaction method according to Claim 37, **characterized in that** the 3' end of the single-stranded DNA molecule has a modification.

39. Polymerase chain reaction method according to Claim 38, **characterized in that** the 3' end of the single-stranded DNA molecule is modified by way of the OH group being replaced with a phosphate group.

40. Polymerase chain reaction method according to any of Claims 37 to 39, **characterized in that** the backbone of the DNA molecule is modified.

41. Polymerase chain reaction method according to Claim 40, **characterized in that** the backbone of the DNA molecule is modified at least at the 3' end between the last and the second-last base.

42. Polymerase chain reaction method according to either of Claims 40 and 41, **characterized in that** the phosphate in the backbone of the DNA molecule is replaced with phosphothioate.

43. Polymerase chain reaction method according to any of Claims 37 to 42, **characterized in that** the oligonucleotide is a circular DNA molecule.

44. Polymerase chain reaction method according to any of Claims 30 to 43, **characterized in that** the ratio of polymerase to target substrate bases is between 1:20 and 1:1000.

45. Polymerase chain reaction method according to any of Claims 30 to 44, **characterized in that** the target substrate used is a set of DNA molecules with various sequence motifs.

46. Polymerase chain reaction method according to any of Claims 30 to 45, **characterized in that** the target substrate forms a DNA double strand at least during the annealing step of the polymerase chain reaction.

47. Polymerase chain reaction method according to Claim 46, **characterized in that** the annealing is carried out at a temperature of between 40 and 70°C.

48. Polymerase chain reaction method according to any of Claims 30 to 47, **characterized in that** the target substrate is up to 10 000 bases in length.

49. Polymerase chain reaction method according to any of Claims 30 to 36, **characterized in that** the 5' end of the double-stranded DNA carries a modification.

50. Polymerase chain reaction method according to Claim 1 or 2, **characterized in that** the target substrate is an RNA.

51. Polymerase chain reaction method according to Claim 50, **characterized in that** the RNA comprises dideoxynucleotides.

52. Polymerase chain reaction method according to Claim 50 or 51, **characterized in that** the 3' end of the RNA has a modification.

53. Polymerase chain reaction method according to Claim 52, **characterized in that** the 3' end of the RNA is modified by way of the OH group being replaced with a phosphate group.

54. Polymerase chain reaction method according to any of Claims 50 to 53, **characterized in that** the backbone of the RNA is modified.

55. Polymerase chain reaction method according to Claim 54, **characterized in that** the backbone of the oligonucleotide is modified at least at the 3' end between the last and the second-last base.

56. Polymerase chain reaction method according to either of Claims 54 and 55, **characterized in that** the phosphate in the backbone of the oligonucleotide is replaced with phosphothioate.

57. Polymerase chain reaction method according to any of Claims 50 to 56, **characterized in that** the RNA consists of a set of RNA molecules with various sequence motifs.

58. Polymerase chain reaction method according to any of Claims 50 to 57, **characterized in that** the RNA target molecule is a homopolymer.

59. Polymerase chain reaction method according to any of Claims 50 to 58, **characterized in that** the target substrate forms a DNA/RNA double strand at least during the annealing step of the polymerase chain reaction.

60. Polymerase chain reaction method according to Claim 59, **characterized in that** the annealing is carried out at a temperature of between 40 and 70°C.

61. Polymerase chain reaction method according to any of Claims 50 to 60, **characterized in that** the target substrate is between 10 and 10 000 bases in length.

62. Polymerase chain reaction method according to any of Claims 50 to 61, **characterized in that** the 5' end of the RNA carries a modification.

63. Kit for carrying out a polymerase chain reaction method according to any of Claims 1 to 62, said kit comprising at least one target substrate and furthermore at least one primer, deoxyribonucleoside triphosphates, a PCR buffer, a solution containing Mg²⁺ ions, PCR additives and/or a polymerase having proofreading activity, wherein the target substrate is a nucleic acid, wherein furthermore the target substrate forms double-stranded structures under polymerase chain reaction conditions, and wherein the target substrate is not amplified, and wherein further the target substrate reduces degradation of the primers and/or the template DNA due to the proofreading activity of the polymerase.

64. Kit according to Claim 63, **characterized in that** the PCR additives are selected from among betaines, polyethylene glycol (PEG), dextran, glycerol, dimethyl sulphoxide (DMSO), bovine serum albumin (BSA), single-stranded DNA-binding protein and/or (non-ionic) detergents.

## Revendications

1. Procédé de réaction en chaîne par polymérase, dans lequel on utilise un acide nucléique de matrice, au moins une amorce, des désoxyribonucléosidetriphosphates, ainsi qu'une polymérase ayant une activité de correction, **caractérisé en ce que**, lors de la réaction en chaîne par polymérase, on ajoute au moins un substrat cible, le substrat cible étant un acide nucléique, le substrat cible formant en outre, dans les conditions de la réaction en chaîne par polymérase, des structures double brin, et le substrat cible ne subissant pas d'amplification, le substrat cible provoquant en outre une réduction de la dégradation de l'amorce et/ou de l'ADN matrice sous l'effet de l'activité de correction de la polymérase.

2. Procédé de réaction en chaîne par polymérase selon la revendication 1, **caractérisé en ce que** l'au moins un substrat cible inhibe en totalité ou en partie l'activité de 3'-5'-exonucléase de la polymérase ayant une activité de correction.

3. Procédé de réaction en chaîne par polymérase selon la revendication 1 ou 2, **caractérisé en ce que** le substrat cible est un oligonucléotide simple brin.

4. Procédé de réaction en chaîne par polymérase selon la revendication 3, **caractérisé en ce que** la non extensibilité de l'oligonucléotide simple brin à son extrémité 3' est provoquée par une modification.

5. Procédé de réaction en chaîne par polymérase selon la revendication 4, **caractérisé en ce que**, en tant que modification à l'extrémité 3' de l'oligonucléotide simple brin, on remplace le groupe OH par un groupe phosphate ou un autre radical, ou on réalise la non extensibilité par un didésoxy-nucléotide, une ou plusieurs base(s) inverse(s), un ARN, des sites abasiques, un espaceur, des colorants, des radicaux désactivateurs, tels que par exemple le Black Hole Quencher, le Dabcyl, le Minor-Groove-Binder, des bases modifiées, telles que par exemple des superbases ou des bases halogénées, ou des analogues de bases appropriées.

6. Procédé de réaction en chaîne par polymérase selon l'une des revendications 3 à 5, **caractérisé en ce que** la colonne vertébrale de l'oligonucléotide est modifiée.

7. Procédé de réaction en chaîne par polymérase selon la revendication 6, **caractérisé en ce que** la colonne vertébrale de l'oligonucléotide est modifiée au moins à l'extrémité 3' entre la dernière et l'avant-dernière base.

8. Procédé de réaction en chaîne par polymérase selon l'une des revendications 6 ou 7, **caractérisé en ce que** le phosphate, dans la colonne vertébrale de l'oligonucléotide, est remplacé par un phosphothioate.

9. Procédé de réaction en chaîne par polymérase selon l'une des revendications 3 à 8, **caractérisé en ce que** l'oligonucléotide est au moins partiellement complémentaire de l'acide nucléique de matrice, de sorte qu'un double brin d'ADN peut se former.

10. Procédé de réaction en chaîne par polymérase selon l'une des revendications 3 à 8, **caractérisé en ce que** l'oligonucléotide comprend une séquence aléatoire.

11. Procédé de réaction en chaîne par polymérase selon l'une des revendications 3 à 10, **caractérisé en ce que** l'oligonucléotide contient des bases choisies dans le groupe consistant en l'adénine (A), la cytosine (C), la guanine (G) et la thymine (T), des bases universelles (telles que par exemple l'inosine, le 3-nitropyrrole ou le 5-nitro-indole), un ou plusieurs uraciles, de la méthylcytosine, des analogues de bases ou des bases modifiées.

12. Procédé de réaction en chaîne par polymérase selon l'une des revendications 3 à 11, **caractérisé en ce que** l'oligonucléotide contient un ou plusieurs PNA (acides nucléiques peptidiques) ou LNA (acides nucléiques verrouillés).

13. Procédé de réaction en chaîne par polymérase selon l'une des revendications 3 à 8 ou 10 à 12, **caractérisé en ce que** l'oligonucléotide est un homo-oligonucléotide.

14. Procédé de réaction en chaîne par polymérase selon l'une des revendications 3 à 13, **caractérisé en ce que** l'oligonucléotide a une longueur de 10 à 100 bases, de préférence de 12 à 80, en particulier de 20 à 45 bases.

15. Procédé de réaction en chaîne par polymérase selon l'une des revendications 3 à 14, **caractérisé en ce que** l'oligonucléotide est présent dans la réaction en chaîne par polymérase à une concentration de 0,1 à 20 M, de préférence de 0,2 à 2,5 M, en particulier de 0,5 à 1,5 M.

16. Procédé de réaction en chaîne par polymérase selon l'une des revendications 3 à 15, **caractérisé en ce que** le rapport en moles de la polymérase ayant une activité de correction au substrat cible est compris entre 1:1 et 1:1000, de préférence entre 1:1 et 1:500 et en particulier entre 1:1 et 1:200.

17. Procédé de réaction en chaîne par polymérase selon la revendication 1 ou 2, **caractérisé en ce qu'**au moins un substrat cible est un oligonucléotide double brin.

18. Procédé de réaction en chaîne par polymérase selon la revendication 17, **caractérisé en ce que** la colonne vertébrale de l'oligonucléotide est modifiée.

19. Procédé de réaction en chaîne par polymérase selon la revendication 18, **caractérisé en ce que** la colonne vertébrale de l'oligonucléotide est modifiée au moins à l'extrémité 3' entre la dernière et l'avant-dernière base.

20. Procédé de réaction en chaîne par polymérase selon l'une des revendications 18 ou 19, **caractérisé en ce que** le phosphate, dans la colonne vertébrale de l'oligonucléotide, est remplacé par un phosphothioate.

21. Procédé de réaction en chaîne par polymérase selon l'une des revendications 17 à 20, **caractérisé en ce que** le substrat cible comprend une séquence cible qui peut former une structure en épingle à cheveu auto-complémentaire d'elle-même.

22. Procédé de réaction en chaîne par polymérase selon la revendication 21, **caractérisé en ce que** le substrat cible comprend au moins une modification dans la séquence de bases et/ou dans la colonne vertébrale de l'ADN.

23. Procédé de réaction en chaîne par polymérase selon l'une des revendications 17 à 22, **caractérisé en ce que** le substrat cible forme, au moins pendant l'étape d'annelage de la réaction en chaîne par polymérase, un double brin d'ADN.

24. Procédé de réaction en chaîne par polymérase selon la revendication 23, **caractérisé en ce que** l'annelage est effectué à une température comprise entre 40 et 70°C.

25. Procédé de réaction en chaîne par polymérase selon l'une des revendications 17 à 24, **caractérisé en ce que**, dans le cas d'une structure en épingle à cheveu du substrat cible, la longueur du domaine auto-complémentaire est comprise entre 10 et 100 bases, de préférence entre 12 et 80 bases, en particulier entre 20 et 45 bases.

26. Procédé de réaction en chaîne par polymérase selon l'une des revendications 17 à 24, **caractérisé en ce que** la longueur du substrat cible est comprise entre 20 et 200 bases, de préférence entre 24 et 160 bases.

27. Procédé de réaction en chaîne par polymérase selon l'une des revendications 17 à 26, **caractérisé en ce que** l'oligonucléotide est présent dans la réaction en chaîne par polymérase à une concentration de 0,05 à 20 M, de préférence de 0,2 à 10 M et en particulier de 0,2 à 2,5 M.

28. Procédé de réaction en chaîne par polymérase selon l'une des revendications 17 à 27, **caractérisé en ce que** le rapport en moles de la polymérase ayant une activité de correction au substrat cible est compris entre 1:0,5 et 1:1000, de préférence entre 1:0,5 et 1:500, et en particulier entre 1:1 et 1:200.

29. Procédé de réaction en chaîne par polymérase selon l'une des revendications 17 à 28, **caractérisé en ce que** l'extrémité 5' de l'oligonucléotide porte une modification.

30. Procédé de réaction en chaîne par polymérase selon la revendication 1 ou 2, **caractérisé en ce que** l'au moins un substrat cible est une molécule d'ADN double brin qui ne contient pas de site de liaison pour l'amorce utilisée lors de la réaction en chaîne par polymérase.

31. Procédé de réaction en chaîne par polymérase selon la revendication 30, **caractérisé en ce que** la molécule d'ADN double brin comprend à son extrémité 3' une modification.

32. Procédé de réaction en chaîne par polymérase selon la revendication 31, **caractérisé en ce que**, en tant que modification à l'extrémité 3' de la molécule d'ADN double brin, le groupe OH est remplacé par un groupe phosphate.

33. Procédé de réaction en chaîne par polymérase selon l'une des revendications 30 à 32, **caractérisé en ce que** la colonne vertébrale de la molécule d'ADN est modifiée.

34. Procédé de réaction en chaîne par polymérase selon la revendication 33, **caractérisé en ce que** la colonne vertébrale de la molécule d'ADN est modifiée au moins à l'extrémité 3' entre la dernière et l'avant-dernière base.

35. Procédé de réaction en chaîne par polymérase selon l'une des revendications 33 ou 34, **caractérisé en ce que** le phosphate, dans la colonne vertébrale de la molécule d'ADN, est remplacé par un phosphothioate.

36. Procédé de réaction en chaîne par polymérase selon l'une des revendications 30 à 35, **caractérisé en ce que** l'oligonucléotide est une molécule d'ADN circulaire.

37. Procédé de réaction en chaîne par polymérase selon la revendication 1 ou 2, **caractérisé en ce que** l'au moins un substrat cible est une molécule d'ADN simple brin qui ne contient pas de site de liaison pour l'amorce utilisée lors de la réaction en chaîne par polymérase.

38. Procédé de réaction en chaîne par polymérase selon la revendication 37, **caractérisé en ce que** la molécule d'ADN simple brin comprend une modification à son extrémité 3'.

39. Procédé de réaction en chaîne par polymérase selon la revendication 38, **caractérisé en ce que**, en tant que modification à l'extrémité 3' de la molécule d'ADN simple brin, le groupe OH est remplacé par un groupe phosphate.

40. Procédé de réaction en chaîne par polymérase selon l'une des revendications 37 à 39, **caractérisé en ce que** la colonne vertébrale de la molécule d'ADN est modifiée.

41. Procédé de réaction en chaîne par polymérase selon la revendication 40, **caractérisé en ce que** la colonne vertébrale de la molécule d'ADN est modifiée au moins à l'extrémité 3' entre la dernière et l'avant-dernière base.

42. Procédé de réaction en chaîne par polymérase selon l'une des revendications 40 ou 41, **caractérisé en ce que** le phosphate, dans la colonne vertébrale de la molécule d'ADN, est remplacé par un phosphothioate.

43. Procédé de réaction en chaîne par polymérase selon l'une des revendications 37 à 42, **caractérisé en ce que** l'oligonucléotide est une molécule d'ADN circulaire.

44. Procédé de réaction en chaîne par polymérase selon l'une des revendications 30 à 43, **caractérisé en ce que** le rapport de la polymérase aux bases du substrat cible est compris entre 1:20 et 1:1000.

45. Procédé de réaction en chaîne par polymérase selon l'une des revendications 30 à 44, **caractérisé en ce qu'**on utilise en tant que substrat cible un ensemble de molécules d'ADN ayant différents motifs de séquences.

46. Procédé de réaction en chaîne par polymérase selon l'une des revendications 30 à 45, **caractérisé en ce que** le substrat cible forme, au moins pendant l'étape d'annelage de la réaction en chaîne par polymérase, un double brin d'ADN.

47. Procédé de réaction en chaîne par polymérase selon la revendication 46, **caractérisé en ce que** l'annelage est réalisé à une température comprise entre 40 et 70°C.

48. Procédé de réaction en chaîne par polymérase selon l'une des revendications 30 à 47, **caractérisé en ce que** la longueur du substrat cible va jusqu'à 10 000 bases.

49. Procédé de réaction en chaîne par polymérase selon l'une des revendications 30 à 36, **caractérisé en ce que** l'extrémité 5' de l'ADN double brin porte une modification.

50. Procédé de réaction en chaîne par polymérase selon la revendication 1 ou 2, **caractérisé en ce que** le substrat cible est un ARN.

51. Procédé de réaction en chaîne par polymérase selon la revendication 50, **caractérisé en ce que** l'ARN contient des didésoxynucléotides.

52. Procédé de réaction en chaîne par polymérase selon la revendication 50 ou 51, **caractérisé en ce que** l'ARN comprend une modification à son extrémité 3'.

53. Procédé de réaction en chaîne par polymérase selon la revendication 52, **caractérisé en ce que**, en tant que modification à l'extrémité 3' de l'ARN, le groupe OH est remplacé par un groupe phosphate.

54. Procédé de réaction en chaîne par polymérase selon l'une des revendications 50 à 53, **caractérisé en ce que** la colonne vertébrale de l'ARN est modifiée.

55. Procédé de réaction en chaîne par polymérase selon la revendication 54, **caractérisé en ce que** la colonne vertébrale de l'oligonucléotide est modifiée au moins à l'extrémité 3' entre la dernière et l'avant-dernière base.

56. Procédé de réaction en chaîne par polymérase selon l'une des revendications 54 ou 55, **caractérisé en ce que** le phosphate, dans la colonne vertébrale de l'oligonucléotide, est remplacé par un phosphothioate.

57. Procédé de réaction en chaîne par polymérase selon l'une des revendications 50 à 56, **caractérisé en ce que** l'ARN est constitué d'un ensemble de molécules d'ARN ayant différents motifs de séquences.

58. Procédé de réaction en chaîne par polymérase selon l'une des revendications 50 à 57, **caractérisé en ce que** la molécule cible d'ARN est un homopolymère.

59. Procédé de réaction en chaîne par polymérase selon l'une des revendications 50 à 58, **caractérisé en ce que** le substrat cible forme, au moins pendant l'étape d'annelage de la réaction en chaîne par polymérase, un double brin d'ADN/ARN.

60. Procédé de réaction en chaîne par polymérase selon la revendication 59, **caractérisé en ce que** l'annelage est effectué à une température comprise entre 40 et 70°C.

61. Procédé de réaction en chaîne par polymérase selon l'une des revendications 50 à 60, **caractérisé en ce que** la longueur du substrat cible est comprise entre 10 et 10 000 bases.

62. Procédé de réaction en chaîne par polymérase selon l'une des revendications 50 à 61, **caractérisé en ce que** l'extrémité 5' de l'ARN porte une modification.

63. Kit pour la mise en oeuvre d'un procédé de réaction en chaîne par polymérase selon l'une des revendications 1 à 62, le kit contenant au moins un substrat cible et en outre au moins une amorce, des désoxyribonucléosidetriphosphates, un tampon pour PCR, une solution contenant des ions Mg²⁺, des additifs pour PCR et/ou une polymérase, ayant une activité de correction, le substrat cible étant un acide nucléique, le substrat cible formant en outre, dans les conditions d'une réaction en chaîne par polymérase, des structures double brin, et le substrat cible ne subissant pas d'amplification, et en outre le substrat cible réduisant la dégradation des amorces et/ou de l'ADN de matrice sous l'effet de l'activité de correction de la polymérase.

64. Kit selon la revendication 63, **caractérisé en ce que** les additifs pour PCR sont choisis parmi la bétaïne, le polyéthylèneglycol (PEG), le dextran, le glycérol, le diméthylsulfoxyde (DMSO), la sérumalbumine bovine (BSA), une protéine de liaison à l'ADN simple brin (single-strand DNA-binding-protein) et/ou des détergents (non ioniques).
